# EUROPEAN PATENT APPLICATION

(11) **EP 4 464 693 A1**
(43) Date of publication of application: **20.11.2024**
(21) Application number: 23740488.4
(22) Date of filing: 13.01.2023
(51) Int. Cl.: C07D 213/16, A61K 31/167, A61K 31/4418, A61K 31/444, A61K 31/4439, A61K 31/506, A61K 31/426, A61K 31/4164, A61P 35/00, C07C 237/20

(54) **NOVEL AMINOBENZENE DERIVATIVE HAVING CANCER CELL GROWTH INHIBITORY EFFECT, AND PREVENTIVE OR THERAPEUTIC PHARMACEUTICAL COMPOSITION CONTAINING SAME AS ACTIVE INGREDIENT**

(30) Priority: 14.01.2022 KR 20220006124
(71) Applicant: Daewoong Pharmaceutical Co., Ltd., Hwaseong-si, Gyeonggi-do 18623 (KR)
(72) Inventor: KANG, Youngku, Seoul 06170 (KR); LEE, Inyoung, Seoul 06170 (KR); YOO, Bora, Seoul 06170 (KR); KIM, Ji Duck, Seoul 06170 (KR); CHOI, Youngwoo, Seoul 06170 (KR); HA, Sujin, Seoul 06170 (KR); KWON, Ahreum, Seoul 06170 (KR); PARK, Joon Seok, Seoul 06170 (KR)
(74) Representative: Germain Maureau
(86) International application number: PCT/KR2023/000625
(87) International publication number: WO 2023/136645

(57) **Abstract**

A compound represented by Chemical Formula 1 or 2 of the present invention, or a pharmaceutically acceptable salt thereof can be used favorably for preventing or treating cancer or tumors.

## Description

### [TECHNICAL FIELD]

The present invention relates to a compound having a novel structure that can be used favorably for preventing or treating cancer or tumors.

### [BACKGROUND]

Hippo signaling pathway affects the size and number of cells constituting an organ, and affects organ development and cell growth. In addition, inappropriate regulation of Hippo signaling pathway has been associated with the development of several cancers (breast cancer, head and neck cancer, colon cancer, ovarian cancer, liver cancer, brain cancer, prostate cancer, mesothelioma, sarcoma, etc.).

The NF2, Mst1/2, and Lats1/2 genes constituting the Hippo signaling system are mutated in many carcinomas. Mutations of such tumor suppressors induce sustained binding of YAP/TAZ and TEAD to induce gene expression associated with cancer cell growth. The Hippo signaling system is composed of several components such as NF2(neurofibromatosis type 2), Mst1/2(mammalian Ste20-like kinases 1 and 2), Lats1/2(large tumor suppressor 1/2), YAP/TAZ(yes-associated protein/WW domain-containing transcription regulator protein), and TEADs(transcriptional enhanced associate domains).

When YAP and TAZ are excessively activated by one or more mutations in the Hippo signaling pathway, it leads to tissue overgrowth and tumor formation, which is inversely correlated with the survival rate of cancer patients. The activation of YAP/TAZ is regulated by the response of a series of tumor suppressor genes that constitute the Hippo signaling pathway. NF2, Mst1/2, and Lats1/2 exist as typical tumor suppressor genes. When phosphorylation of Lats1/2 by Mst1/2 is mediated through activation of NF2 which is the highest molecule, phosphorylation of YAP and TAZ existing in the cytoplasm are promoted. The phosphorylated YAP and TAZ undergoes a ubiquitination process and are then decomposed by proteasome. Therefore, when the Hippo signaling system is turned on, YAP and TAZ are turned off. In contrast, under conditions in which tumor suppressor genes are turned off, i.e. when Hippo signaling is turned off, YAP and TAZ are turned on and translocate to the nucleus, which binds to four proteins of the TEAD family (TEAD1/2/3/4) and induces the expression of target genes such as CTGF (connective tissue growth factor), CYR61 (cysteine-rich angiogenic inducer 61), Gli2 (GLI family zinc finger 2), Birc2/5(Baculoviral IAP repeat containing 2/5), FGF(fibroblast growth factor). In this manner, genes turned on by the YAP/TAZ-transcription factor complex regulate cell growth, cell migration, and apoptosis.

Abnormalities in the Hippo signaling system have been found in several carcinomas, and several research results have been reported to develop anticancer drugs targeting YAP-TEAD. Furthermore, the relevance of the Hippo signaling system has been revealed in the process of acquiring resistance after application of existing approved anticancer drugs, and several studies are underway to demonstrate the potential of YAP-TEAD anticancer drugs as combination therapeutics to treat drug resistance.

Therefore, there is a need to develop a small molecule inhibitor for treating cancer caused by dysregulation of the Hippo signaling system.

In this regard, the present inventors have studied medicines that can be used favorably for treating cancer in which the regulation of Hippo signaling is turned off, and as a result, found that the YAP-TEAD inhibitor according to the present invention described hereinafter binds to the palmitate binding site that mediates TEAD palmitoylation, and also inhibits the in vitro growth of a Hippo pathway mutated cell line. Through this, it has been found that the YAP-TEAD inhibitor according to the present invention can be used favorably for treating cancer or tumors in which Hippo signaling is turned off, and completed the present invention.

### [DETAILED DESCRIPTION OF THE INVENTION]

### [Technical Problem]

It is an object of the present invention to provide a compound having a novel structure that can be used favorably for preventing or treating cancer or tumors.

### [Technical Solution]

In order to achieve the above object, provided herein is a compound represented by the following Chemical Formula 1 or 2, or a pharmaceutically acceptable salt thereof: 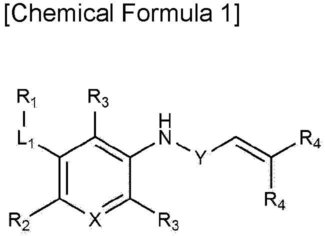 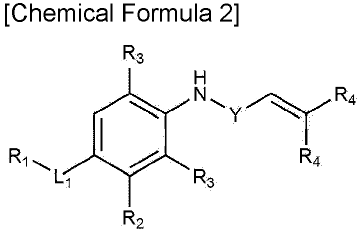
in Chemical Formulas 1 and 2,
L₁ is a single bond, a C₁₋₆ alkylene, a C₂₋₄ alkenylene, or a C₂₋₄ alkynylene,
R₁ is phenyl, or a 5- or 6-membered heterocycle containing 1 to 4 heteroatoms each independently selected from the group consisting of N, O and S,
   the R₁ is unsubstituted or substituted with halogen, a C₁₋₄ alkyl, a C₁₋₄ thioalkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, a C₃₋₆ cycloalkyl, amino, nitro, cyano, a (C₁₋₄ alkyl)amino, or a di(C₁₋₄ alkyl)amino,
R₂ is -N(R₉)-L₂-R₅, 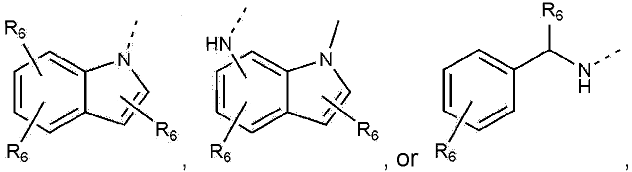
   L₂ is a single bond, a C₁₋₆ alkylene, a C₂₋₄ alkenylene, or a C₂₋₄ alkynylene,
   R₅ is a C₃₋₇ cycloalkyl, phenyl, or a 5- or 6-membered heteroaryl containing 1 to 4 heteroatoms each independently selected from the group consisting of N, O and S,
      the R₅ is unsubstituted or substituted with 1 to 3 substituents each independently selected from the group consisting of hydroxy, halogen, a C₁₋₄ alkyl, a C₁₋₄ alkoxy, a C₁₋₄ haloalkyl, a C₁₋₄ haloalkoxy, a C₁₋₄ thioalkyl, and a C₃₋₆ cycloalkyl,
   each R₆ is independently hydrogen, halogen, a C₁₋₆ alkyl, a C₁₋₆ haloalkyl, a C₁₋₆ alkoxy, or a C₃₋₇ cycloalkyl,
   R₉ is hydrogen, halogen, a C₁₋₄ alkyl, a C₁₋₄ alkoxy, a C₁₋₄ haloalkyl, a C₁₋₄ haloalkoxy, or a C₃₋₆ cycloalkyl,
each Rs is independently hydrogen, halogen, a C₁₋₆ alkyl, a C₁₋₆ haloalkyl, or a C₁₋₄ alkoxy,
each R₄ is independently hydrogen, a C₁₋₄ alkyl, -CH₂N(R₇)₂, or a 5- or 6-membered heteroaliphatic ring containing one or two N which is substituted with R₇,
   each R₇ is independently hydrogen, or a C₁₋₄ alkyl,
X is CRs, or N,
   R₈ is hydrogen, or halogen, and
Y is CO, CS, or SO₂.

Preferably, L₁ is a single bond, or -CH=CH-.

Preferably, R₁ is phenyl, pyridinyl, pyrimidinyl, imidazolyl, pyrazolyl, triazolyl, tetrazolyl, oxadiazolyl, pyrrolidinyl, imidazolyl, pyrazolyl, oxazolyl, isoxazolyl, thiazolyl, or isothiazolyl,
wherein the R₁ is unsubstituted or substituted with halogen, a C₁₋₄ alkyl, a C₁₋athioalkyl, a C₁₋₄ haloalkyl, a C₁₋₄ alkoxy, a C₁₋₄ haloalkoxy, amino, nitro, cyano, a (C₁₋₄ alkyl)amino, or a di(C₁₋₄ alkyl)amino.

Preferably, L₂ is a single bond, methylene (-CH₂-), or ethylene (-CH₂-CH₂-).

Preferably, R₅ is cyclopentyl, cyclohexyl, phenyl, pyridinyl, pyrimidinyl, or thiazolyl,
wherein the R₅ is unsubstituted or substituted with 1 to 3 substituents each independently selected from the group consisting of hydroxy, halogen, a C₁₋₄ alkyl, a C₁₋₄ alkoxy, a C₁₋₄ haloalkyl, a C₁₋₄ haloalkoxy, a C₁₋₄ thioalkyl, and a C₃₋₆ cycloalkyl.

Preferably, each R₆ is independently hydrogen, fluoro, chloro, difluoromethyl, or trifluoromethyl.

Preferably, R₉ is hydrogen.

Preferably, each R₃ is independently hydrogen, or methoxy.

Preferably, R₄ is all hydrogen, or
one of R₄ is hydrogen, and the other is -CH₂N(R₇)₂, or a 5- or 6-membered heteroaliphatic ring containing one or two N which is substituted with R₇.

Preferably, X is CH, CF, or N.

Preferably, the compound represented by Chemical Formula 1 is a compound represented by the following Chemical Formula 3: 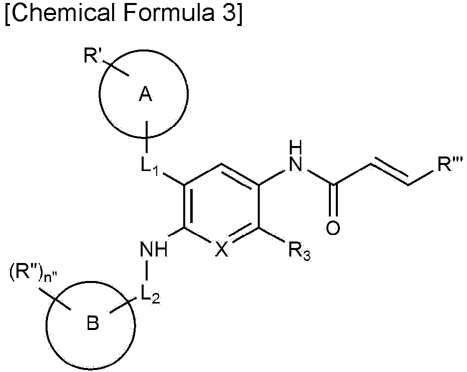
in Chemical Formula 3,
X is CH, CF, or N,
A is benzene, pyridine, pyrimidine, imidazole, pyrazole, triazole, tetrazole, or oxadiazole,
R' is hydrogen, halogen, a C₁₋₄ alkyl, or a C₁₋₄ alkoxy,
L₁ is a single bond, a C₁₋₆ alkylene, or a C₂₋₄ alkenylene,
B is benzene, pyridine, pyrimidine, thiazolyl, cyclopentyl, or cyclohexyl,
each R" is independently hydrogen, hydroxy, halogen, cyano, a C₁₋₄ alkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, or a C₃₋₆ cycloalkyl,
n" is an integer of 1 to 3,
L₂ is a single bond, or a C₁₋₆ alkylene,
R‴ is hydrogen, or -CH₂-N(CH₃)₂, and
R₃ is hydrogen or a C₁₋₄ alkoxy.

Preferably, the compound represented by Chemical Formula 1 is a compound represented by the following Chemical Formula 4: 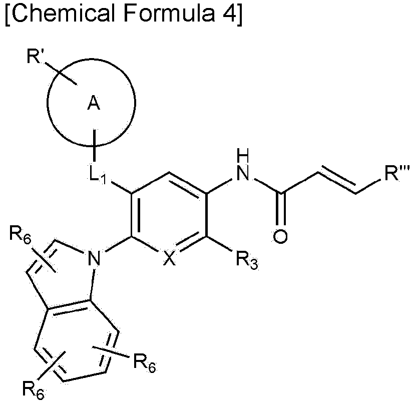
in Chemical Formula 4,
X is CH, CF, or N,
A is benzene, pyridine, pyrimidine, imidazole, pyrazole, triazole, tetrazole, or oxadiazole,
R' is hydrogen, halogen, a C₁₋₄ alkyl, or a C₁₋₄ alkoxy,
L₁ is a single bond, a C₁₋₆ alkylene, or a C₂₋₄ alkenylene,
each R₆ is independently hydrogen, halogen, a C₁₋₆ alkyl, a C₁₋₆ haloalkyl, a C₁₋₆ alkoxy, or a C₃₋₇ cycloalkyl,
R₃ is hydrogen or a C₁₋₄ alkoxy, and
R‴ is hydrogen, or -CH₂-N(CH₃)₂.

Representative examples of the compounds represented by Chemical Formula 1 or Chemical Formula 2 are as follows:
1) N-(6-(cyclohexylamino)-[1,1'-biphenyl]-3-yl)acrylamide,
2) N-(4-(cyclohexylamino)-3-(pyridin-2-yl)phenyl)acrylamide,
3) N-(3-(pyridin-2-yl)-4-((cis-4-(trifluoromethyl)cyclohexyl)amino)phenyl)acrylamide,
4) N-(3-(pyridin-2-yl)-4-((5-(trifluoromethyl)pyridin-2-yl)amino)phenyl)acrylamide,
5) N-(2-methoxy-5-(pyridin-2-yl)-4-((5-(trifluoromethyl)pyridin-2-yl)amino)phenyl)acrylamide,
6) N-(3-(1-methyl-1H-imidazol-4-yl)-4-((5-(trifluoromethyl)pyridin-2-yl)amino)phenyl)acrylamide,
7) N-(4-((3-chloro-5-(trifluoromethyl)pyridin-2-yl)amino)-3-(pyridin-2-yl)phenyl)acrylam ide,
8) N-(4-((4,5-dichloropyridin-2-yl)amino)-3-(pyridin-2-yl)phenyl)acrylam ide,
9) N-(4-((3-chloro-5-(trifluoromethyl)pyridin-2-yl)amino)-3-(1 -methyl-1H-imidazol-4-yl)phenyl)acrylamide ,
10) N-(4-((4,5-dichloropyridin-2-yl)amino)-3-(1-methyl-1H-imidazol-4-yl)phenyl)acrylam ide,
11) N-(6-((4-fluorobenzyl)amino)-[1,1'-biphenyl]-3-yl)acrylamide,
12) N-(4-((4-fluorobenzyl)amino)-3-(pyridin-2-yl)phenyl)acrylamide,
13) N-(3-(5-chloropyridin-2-yl)-4-((5-(trifluoromethyl)pyridin-2-yl)amino)phenyl)acrylamide,
14) N-(4-(pyridin-2-yl)-3-((4-(trifluoromethyl)phenyl)amino)phenyl)acrylamide,
15) N-(3-(pyrimidin-4-yl)-4-((5-(trifluoromethyl)pyridin-2-yl)amino)phenyl)acrylamide,
16) N-(4-((4,5-dichloropyridin-2-yl)amino)-3-(pyrimidin-4-yl)phenyl)acrylamide,
17) N-(3-(pyridin-4-yl)-4-((5-(trifluoromethyl)pyridin-2-yl)amino)phenyl)acrylamide,
18) N-(4-((4,5-dichloropyridin-2-yl)amino)-3-(pyridin-4-yl)phenyl)acrylam ide,
19) N-(4-(5-chloro-4-fluoro-1H-indol-1-yl)-3-(pyridin-2-yl)phenyl)acrylam ide,
20) N-(4-((5-cyclopropylpyridin-2-yl)amino)-3-(pyridin-2-yl)phenyl)acrylam ide,
21) N-(4-((3-fluoro-5-(trifluoromethyl)pyridin-2-yl)amino)-3-(pyridin-2-yl)phenyl)acrylam ide,
22) N-(6-(((1r,4r)-4-hydroxycyclohexyl)amino)-5-phenylpyridin-3-yl)acrylamide,
23) (E)-N-(3-(4-fluorostyryl)-4-((2-(thiazol-2-yl)ethyl)amino)phenyl)acrylamide,
24) (E)-N-(3-(4-fluorostyryl)-4-((2-(thiazol-2-yl)ethyl)amino)phenyl)ethanesulfonamide,
25) N-(4-(cyclohexylamino)-3-(pyridin-3-yl)phenyl)acrylamide,
26) N-(4-(cyclopentylamino)-3-(pyridin-2-yl)phenyl)acrylamide,
27) (E)-N-(5-(4-fluorostyryl)-6-((2-(thiazol-2-yl)ethyl)amino)pyridin-3-yl)acrylamide,
28) (E)-N-(5-(4-fluorostyryl)-6-((2-(thiazol-2-yl)ethyl)amino)pyridin-3-yl)ethanesulfonamide,
29) N-(3-fluoro-5-(pyridin-2-yl)-4-((5-(trifluoromethyl)pyridin-2-yl)amino)phenyl)acrylamide,
30) N-(4-((5-chloro-4-cyclopropylpyridin-2-yl)amino)-3-(pyridin-2-yl)phenyl)acrylam ide,
31) N-(3-(1-methyl-1H-imidazol-4-yl)-4-((4-(trifluoromethyl)pyridin-2-yl)amino)phenyl)acrylamide,
32) (E)-4-(dimethylamino)-N-(3-(1-methyl-1H-pyrazol-3-yl)-4-((4-(trifluoromethyl)pyridin-2-yl)amino)phenyl)-2-butenamide,
33) N-(4-((5-isopropylpyridin-2-yl)amino)-3-(pyridin-2-yl)phenyl)acrylamide,
34) N-(4-((5-ethynylpyridin-2-yl)amino)-3-(1 -methyl-1H-pyrazol-3-yl)phenyl)acrylam ide,
35) N-(3-(1-methyl-1H-imidazol-4-yl)-4-((4-(trifluoromethyl)phenyl)amino)phenyl)acrylamide,
36) N-(4-((3-fluoro-5-(trifluoromethyl)pyridin-2-yl)amino)-3-(1 -methyl-1Himidazol-4-yl)phenyl)acrylamide,
37) N-(4-((4-chloro-5-(trifluoromethyl)pyridin-2-yl)amino)-3-(1-methyl-1H-pyrazol-3-yl)phenyl)acrylamide ,
38) N-(3-fluoro-5-(1-methyl-1H-imidazol-4-yl)-4-((5-(trifluoromethyl)pyridin-2-yl)amino)phenyl)acrylamide,
39) N-(2'-((5-(trifluoromethyl)pyridin-2-yl)amino)-[2,3'-bipyridin]-5'-yl)acrylamide,
40) N-(4-((3-fluoro-4-(trifluoromethyl)phenyl)amino)-3-(1 -methyl-1Himidazol-4-yl)phenyl)acrylamide,
41) N-(5-(1-methyl-1H-imidazol-4-yl)-6-((4-(trifluoromethyl)phenyl)amino)pyridin-3-yl)acrylamide,
42) N-(3-(1-methyl-1H-pyrazol-3-yl)-4-((4-(trifluoromethyl)phenyl)amino)phenyl)acrylamide,
43) N-(4-((3-chloro-4-(trifluoromethyl)phenyl)amino)-3-(1 -methyl-1Himidazol-4-yl)phenyl)acrylamide,
44) N-(4-((2,3-difluoro-4-(trifluoromethyl)phenyl)amino)-3-(1 -methyl-1H-imidazol-4-yl)phenyl)acrylamide,
45) N-(3-(1-methyl-1H-imidazol-4-yl)-4-((4-(trifluoromethoxy)phenyl)amino)phenyl)acrylamide,
46) N-(4-((3,5-difluoro-4-(trifluoromethyl)phenyl)amino)-3-(1 -methyl-1Himidazol-4-yl)phenyl)acrylamide,
47) N-(4-((4-chlorophenyl)amino)-3-(1-methyl-1H-imidazol-4-yl)phenyl)acrylam ide,
48) N-(4-((5-bromo-6-methylpyridin-2-yl)amino)-3-(1-methyl-1H-imidazol-4-yl)phenyl)acrylamide,
49) N-(4-((5-chloro-6-methylpyridin-2-yl)amino)-3-(1-methyl-1H-imidazol-4-yl)phenyl)acrylamide,
50) N-(4-((5-chloropyridin-2-yl)amino)-3-(1-methyl-1H-imidazol-4-yl)phenyl)acrylam ide,
51) N-(4-((5-bromo-4-chloropyridin-2-yl)amino)-3-(1-methyl-1H-imidazol-4-yl)phenyl)acrylamide,
52) N-(4-((5-bromopyridin-2-yl)amino)-3-(1-methyl-1H-imidazol-4-yl)phenyl)acrylam ide,
53) N-(4-((3-chloro-5-(trifluoromethyl)pyridin-2-yl)amino)-3-(1 -methyl-1H-pyrazol-3-yl)phenyl)acrylamide,
54) N-(3-(1-methyl-1H-pyrazol-3-yl)-4-((4-(trifluoromethyl)pyridin-2-yl)amino)phenyl)acrylamide,
55) N-(4-((4,5-dichloropyridin-2-yl)amino)-3-(1-methyl-1H-pyrazol-3-yl)phenyl)acrylamide, and
56) N-(4-((3-fluoro-5-(trifluoromethyl)pyridin-2-yl)amino)-3-(1 -methyl-1H-pyrazol-3-yl)phenyl)acrylamide.

In addition, as an example, the compound represented by Chemical Formula 1 according to the present invention can be prepared as shown in the following Reaction Scheme 1, and the compound represented by Chemical Formula 2 according to the present invention can be prepared as shown in the following Reaction Scheme 2: 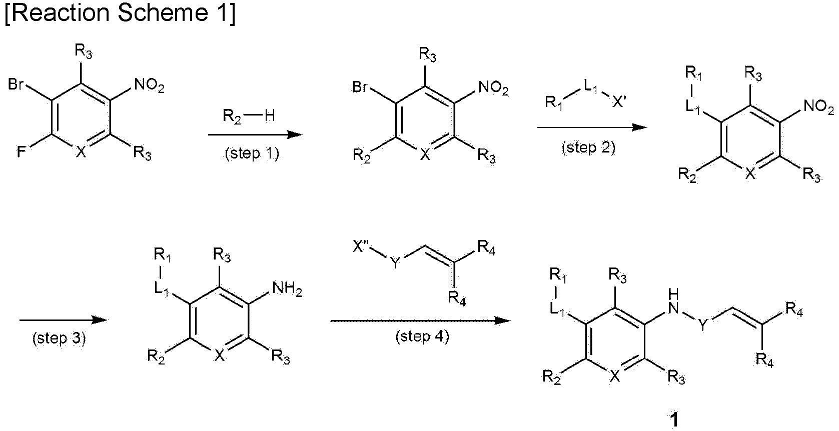 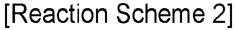 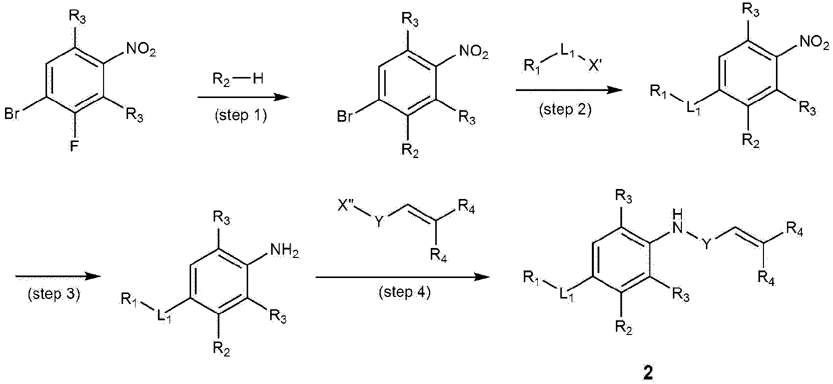 in Reaction Schemes 1 and 2, the remaining substituents except for X' and X" are the same as defined above, and X' and X" are explained below.

Each step 1 of Reaction Schemes 1 and 2 is an amine substitution reaction, and each step 2 is a Suzuki coupling reaction, wherein the order of step 1 and step 2 may be changed depending on the reactivity of each reactant. At this time, X' is a substituent for the Suzuki coupling reaction, and may be -B(OH)₂, tributylstannyl, and the like, but is not limited thereto.

Each step 3 of Reaction Schemes 1 and 2 is a reaction of reducing a nitro group, and the reaction may be performed using a palladium catalyst in the presence of hydrogen, but is not limited thereto.

Each step 4 of Reaction Schemes 1 and 2 is a reaction of an amine group with a carbonyl group or a sulfonyl group, and X" may be hydroxy or halogen (e.g., chloro), but is not limited thereto.

The preparation method may be more specifically described in the Example Examples described hereinafter.

Further provided is a pharmaceutical composition for preventing or treating cancer or tumors, comprising the compound represented by Chemical Formula 1 or 2, or a pharmaceutically acceptable salt thereof as an active ingredient.

As used herein, the term "prevention" refers to any act to delay or inhibit occurrence, spread or recurrence of the above-mentioned diseases by administration of the composition of the present invention, and "treatment" refers to any act to improve or change the symptoms of the above diseases for the better by administration of the composition of the present invention.

### [Advantageous Effects]

A compound represented by Chemical Formula 1 or 2 of the present invention, or a pharmaceutically acceptable salt thereof can be used favorably for preventing or treating cancer or tumors.

### [DETAILED DESCRIPTION OF THE EMBODIMENTS]

Hereinafter, preferred examples are presented to assist in the understanding of the present invention. However, the following examples are for illustrative purposes only, and should not be construed as limiting the scope of the present invention to these examples.

### Example 1: Preparation of N-(6-(cyclohexylamino)-[1,1'-biphenyl]-3-yl)acrylamide

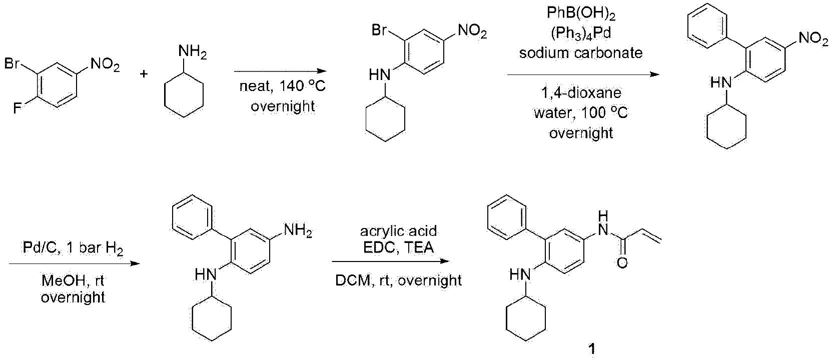

### (Step 1)

2-Bromo-1-fluoro-4-nitrobenzene (5.0 mmol, 1.1 g, 1.0 eq) was dissolved in cyclohexanamine (1.5 mL) and then allowed to react at 140°C overnight. After the reaction was completed, the temperature was cooled to room temperature. Water (30 mL) was added thereto, stirred for 1 hour, and the resulting solid was filtered to give 2-bromo-N-cyclohexyl-4-nitroaniline (1.3 g, yield: 86%).

### (Step 2)

In a sealed tube, 2-bromo-N-cyclohexyl-4-nitroaniline (1.0 mmol, 0.29 g, 1.0 eq) was dissolved in a mixed solution (2.2 mL) of 1,4-dioxane and water (1,4-dioxane:water = 10:1 (v:v)). Phenylboronic acid (1.2 mmol, 0.15 g, 1.2 eq), sodium carbonate (2.0 mmol, 0.21 g, 2.0 eq), and (Ph₃P)₄Pd (0.05 mmol, 0.057 g, 0.05 eq) was sequentially added thereto, and allowed to react at 100°C overnight. After the reaction was completed, the solvent was removed, and the resulting material was isolated and purified by column chromatography (ethyl acetate:hexane = 1:10 (v:v)) to give N-cyclohexyl-5-nitro-[1,1'-biphenyl]-2-amine (0.24 g, yield: 81%).

### (Step 3)

N-cyclohexyl-5-nitro-[1,1'-biphenyl]-2-amine (0.8 mmol, 0.24 g, 1.0 eq) was dissolved in methanol (10 mL), and then Pd/C (24 mg) was added thereto and allowed to react overnight at room temperature under hydrogen (H₂) atmosphere at one atm. After the reaction was completed, the reaction mixture was filtered through a celite pad, and the filtrate was concentrated to give N²-cyclohexyl-[1,1'-biphenyl]-2,5-diamine (0.24 g, yield: 100%).

### (Step 4)

N²-cyclohexyl-[1,1'-biphenyl]-2,5-diamine (0.1 mmol, 0.026 g, 1.0 eq), acrylic acid (0.12 mmol, 0.0086 g, 1.2 eq), N-(3-dimethylaminopropyl)-N-ethylcarbodiimide hydrochloride (0.2 g), and triethylamine (0.1 mL) were dissolved in dichloromethane (1 mL), and allowed to react overnight at room temperature. After the reaction was completed, dichloromethane was removed, and the resulting material was purified by column chromatography to give Compound 1 (0.011 g, yield: 35%).

¹H NMR (500 MHz, CDCl₃) δ 7.50-7.30 (m, 8H), 7.27-7.22 (m, 1H), 6.66 (d, J = 8.8 Hz, 1H), 6.36 (d, J = 16.9 Hz, 1H), 6.23 (d, J = 10.2 Hz, 1H), 5.70-5.63 (m, 1H), 3.82 (brs, 1H), 3.30-3.20 (m, 1H), 2.02-1.91 (m, 2H), 1.68-1.64 (m, 3H), 1.36-1.30 (m, 2H), 1.17 (td, J = 11.7, 3.8 Hz, 1H), 1.10-1.02 (m, 2H).

### Example 2: Preparation of N-(4-(cyclohexylamino)-3-(pyridin-2-yl)phenyl)acrylamide

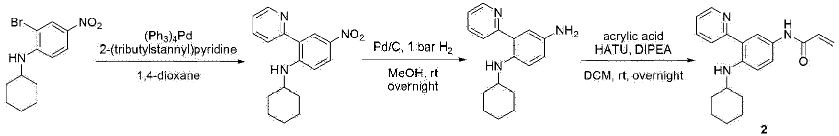

### (Step 1)

In a sealed tube, 2-bromo-N-cyclohexyl-4-nitroaniline (0.5 mmol, 0.15 g, 1.0 eq) was dissolved in 1,4-dioxane (1 mL), and then 2-(tributylstannyl)pyridine (0.6 mmol, 0.2 mL, 1.2 eq) and (Ph₃P)₄Pd (0.05 mmol, 0.057 g, 0.05 eq) were sequentially added thereto, and allowed to react at 150°C overnight. After the reaction was completed, the solvent was removed, and the resulting material was isolated and purified by column chromatography (ethyl acetate:hexane = 1:5 (v:v)) to give N-cyclohexyl-4-nitro-2-(pyridin-2-yl)aniline (0.1 g, yield: 67%).

### (Step 2)

N-cyclohexyl-4-nitro-2-(pyridin-2-yl)aniline (1.0 mmol, 0.29 g, 1.0 eq) was dissolved in methanol (10 mL), and then Pd/C (30 mg) was added thereto and allowed to react overnight at room temperature under hydrogen (H₂) atmosphere at one 1 atm. After the reaction was completed, the reaction mixture was filtered through a celite pad, and the filtrate was concentrated to give N¹-cyclohexyl-2-(pyridin-2-yl)benzene-1,4-diamine (0.27 g, yield: 100%).

### (Step 3)

N¹-cyclohexyl-2-(pyridin-2-yl)benzene-1,4-diamine (0.1 mmol, 0.027 g, 1.0 eq), acrylic acid (0.12 mmol, 0.0086 g, 1.2 eq), 1-[bis(dimethylamino)methylene]-1H-1,2,3-triazolo[4,5-b]pyridinium 3-oxide hexafluorophosphate (0.091 g), and N,N-diisopropylethylamine (0.1 mL) were dissolved in dichloromethane (1 mL), and allowed to react overnight at room temperature. After the reaction was completed, dichloromethane was removed, and the resulting material was purified by column chromatography to give Compound 2 (13 mg, yield: 42%).

¹H NMR (500 MHz, CDCl₃) δ 8.57 (d, J = 4.6 Hz, 1H), 7.94 (d, J = 2.3 Hz, 1H), 7.72-7.68 (m, 2H), 7.28 (d, J = 2.1 Hz, 1H), 7.16 (dd, J = 8.5, 3.1 Hz, 1H), 6.72 (d, J = 8.9 Hz, 1H), 6.40 (d, J = 16.8 Hz, 2H), 6.23 (dd, J = 16.8, 10.2 Hz, 1H), 5.71 (d, J = 10.2 Hz, 1H), 3.45-3.43 (m, 1H), 2.06-1.97 (m, 2H), 1.80-1.70 (m, 2H), 1.64-1.57 (m, 1H) 1.45-1.23 (m, 5H).

### Example 3: Preparation of N-(3-(pyridin-2-yl)-4-((cis-4-(trifluoromethyl)cyclohexyl)amino)phenyl)acrylamide

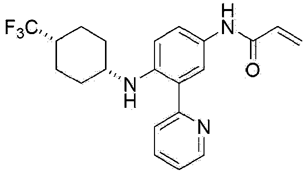

The title compound (6 mg, yield: 15%) was obtained in the same manner as in Example 1, except that cis-4-(trifluoromethyl)-cyclohexan-1-amine was used instead of cyclohexylamine in step 1 of Example 1, and 2-tributylstannylpyridine was used instead of phenylboronic acid in step 2 of Example 1.

¹H NMR (500 MHz, CDCl₃) δ 8.52 (d, J = 4.3 Hz, 1H), 7.99 (d, J = 1.4 Hz, 1H), 7.77-7.62 (m, 4H), 7.29 (dd, J = 8.7, 1.6 Hz, 1H), 7.20-7.10 (m, 1H), 6.69 (s, 1H), 6.39 (dd, J = 17.2, 4.8 Hz, 1H), 6.27 (dd, J = 16.8, 10.1 Hz, 1H), 5.68 (d, J = 10.1 Hz, 1H), 3.35-3.20 (m, 1H), 2.27-2.23 (m, 2H), 2.15-1.98 (m, 2H), 1.50-1.38 (m, 2H), 1.25-1.20 (m, 2H).

### Example 4: Preparation of N-(3-(pyridin-2-yl)-4-((5-(trifluoromethyl)pyridin-2-yl)amino)phenyl)acrylamide

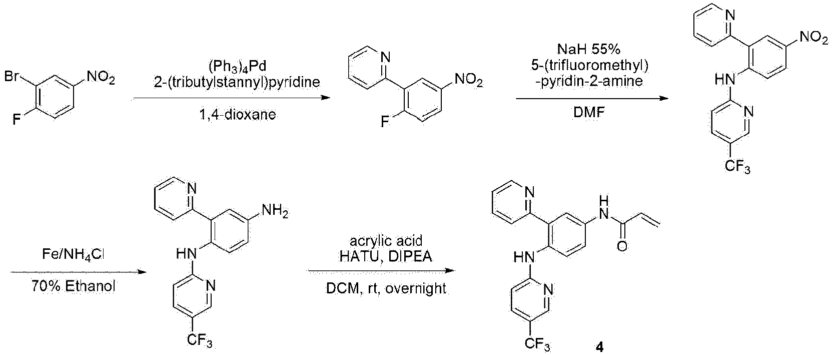

### (Step 1)

In a sealed tube, 2-bromo-1-fluoro-4-nitrobenzene (4.54 mmol, 1.0 g, 1.0 eq) was dissolved in 1,4-dioxane (20 mL), and then 2-(tributylstannyl)pyridine (5.0 mmol, 1.84 g, 1.1 eq) and (Ph₃P)₄Pd (0.45 mmol, 0.52 g, 0.1 eq) were sequentially added thereto and allowed to react at 150°C overnight. After the reaction was completed, the solvent was removed, and the resulting material was isolated and purified by column chromatography (ethyl acetate:hexane = 1:3 (v:v)) to give 2-(2-fluoro-5-nitrophenyl)pyridine (0.68 g, yield: 68%).

### (Step 2)

2-(2-Fluoro-5-nitrophenyl)pyridine (3.7 mmol, 0.599 g, 1.2 eq) was dissolved in dimethylformamide (10 mL), and then 55% NaH (10.0 mmol, 0.43 g) was added thereto and stirred for 10 minutes. 5-(Trifluoromethyl)pyridin-2-amine (3.1 mmol, 0.68 g, 1.0 eq) was added thereto, and stirred at room temperature for 2 hours. When the reaction was completed, the reaction mixture was cooled to 0°C, and then distilled water (30 mL) was added. The resulting solid was filtered to give N-(4-nitro-2-(pyridin-2-yl)phenyl)-5-(trifluoromethyl)pyridin-2-amine (0.64 g, yield: 57%).

### (Step 3)

N-(4-nitro-2-(pyridin-2-yl)phenyl)-5-(trifluoromethyl)pyridin-2-amine (1.77 mmol, 0.64 g), Fe (17.7 mmol, 0.99 g), NH₄Cl (1.77 mmol, 0.094 g) and 70% ethanol solution (10 mL) were added sequentially to a flask, and then allowed to react overnight at room temperature. After the reaction was completed, Fe and NH₄Cl were removed to give 2-(pyridin-2-yl)-N¹-(5-(trifluoromethyl)pyridin-2-yl)benzene-1,4-diamine (0.62 g, yield: 100%).

### (Step 4)

2-(Pyridin-2-yl)-N¹-(5-(trifluoromethyl)pyridin-2-yl)benzene-1,4-diamine (0.1 mmol, 0.033 g, 1.0 eq), acrylic acid (0.12 mmol, 0.0086 g, 1.2 eq), 1-[bis(dimethylamino)methylene]-1H-1,2,3-triazolo[4,5-b]pyridinium 3-oxide hexafluorophosphate (HATU; 0.12 mmol, 0.0046 g), and N,N-diisopropylethylamine (0.3 mmol, 0.052 mL) were dissolved in dichloromethane (1 mL), and allowed to react overnight at room temperature. After the reaction was completed, dichloromethane was removed, and the resulting material was purified by column chromatography to give Compound 4 (8 mg, 20%).

¹H NMR (500 MHz, DMSO) δ 11.42 (s, 1H), 8.69 (d, J = 4.8 Hz, 1H), 8.44 (s, 1H), 8.31-8.26 (m, 2H), 7.85-7.75 (m, 2H), 7.62 (dd, J = 8.7, 2.1 Hz, 1H), 7.41-7.32 (m, 1H), 7.32-7.26 (m, 2H), 6.82 (d, J = 8.8 Hz, 1H), 6.47 (d, J = 16.6 Hz, 1H), 6.27 (dd, J = 16.8, 10.2 Hz, 1H), 5.80 (d, J = 10.3 Hz, 1H).

### Example 5: Preparation of N-(2-methoxy-5-(pyridin-2-yl)-4-((5-(trifluoromethyl)pyridin-2-yl)amino)phenyl)acrylamide

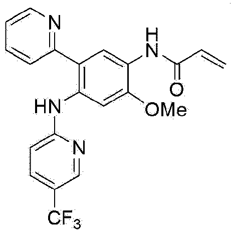

The title compound (6 mg, yield: 15%) was obtained in the same manner as in Example 4, except that 1-bromo-2-fluoro-4-methoxy-5-nitrobenzene was used instead of 2-bromo-1-fluoro-4-nitrobenzene in step 1 of Example 4.

¹H NMR (500 MHz, CDCl₃) δ 12.37 (s, 1H), 8.97 (s, 1H), 8.60 (d, J = 4.3 Hz, 1H), 8.45 (s, 1H), 8.36 (s, 1H), 7.93-7.70 (m, 3H), 7.62 (dd, J = 8.7, 2.3 Hz, 1H), 7.19 (dd, J = 6.6, 5.3 Hz, 1H), 6.79 (d, J = 8.7 Hz, 1H), 6.44 (dd, J = 16.8, 1.0 Hz, 1H), 6.31 (dd, J = 16.8, 10.1 Hz, 1H), 5.76 (dd, J = 10.1, 0.9 Hz, 1H), 3.98 (s, 3H).

### Example 6: Preparation of N-(3-(1-methyl-1H-imidazol-4-yl)-4-((5-(trifluoromethyl)pyridin-2-yl)amino)phenyl)acrylamide

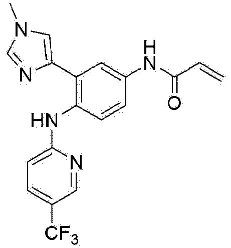

The title compound (8 mg, yield: 20%) was obtained in the same manner as in Example 4, except that 1-methyl-4-(tributylstannyl)-1H-imidazole was used instead of 2-(tributylstannyl)pyridine in step 1 of Example 4.

¹H NMR (500 MHz, MeOD) δ 8.34 (s, 1H), 8.07-7.92 (m, 2H), 7.72-7.66 (m, 2H), 7.73-7.65 (m, 1H), 7.37 (brs, 1H), 6.79 (d, J = 9.0 Hz, 1H), 6.50-6.32 (m, 2H), 5.77 (dd, J = 9.9, 1.9 Hz, 1H), 3.74 (s, 3H).

### Example 7: Preparation of N-(4-((3-chloro-5-(trifluoromethyl)pyridin-2-yl)amino)-3-(pyridin-2-yl)phenyl)acrylamide

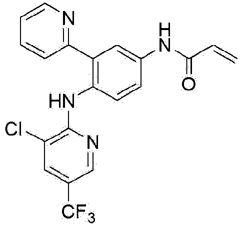

The title compound (20 mg, yield: 47%) was obtained in the same manner as in Example 4, except that 3-chloro-5-(trifluoromethyl)pyridin-2-amine was used instead of 5-(trifluoromethyl)pyridin-2-amine in step 2 of Example 4.

¹H NMR (500 MHz, MeOD) δ 8.67 (d, J = 4.0 Hz, 1H), 8.58 (d, J = 9.0 Hz, 1H), 8.31 (s, 1H), 8.18 (d, J = 2.4 Hz, 1H), 7.99-7.77 (m, 3H), 7.57 (dd, J = 9.0, 2.4 Hz, 1H), 7.36 (dd, J = 6.9, 5.0 Hz, 1H), 6.50-6.35 (m, 2H), 5.78 (dd, J = 9.8, 1.9 Hz, 1H).

### Example 8: Preparation of N-(4-((4,5-dichloropyridin-2-yl)amino)-3-(pyridin-2-yl)phenyl)acrylamide

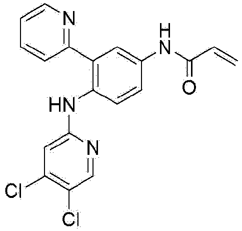

The title compound (14 mg, yield: 36%) was obtained in the same manner as in Example 4, except that 4,5-dichloro-2-amine was used instead of 5-(trifluoromethyl)pyridin-2-amine in step 2 of Example 4.

¹H NMR (500 MHz, MeOD) δ 8.64 (d, J = 4.3 Hz, 1H), 8.07-7.01 (m, 2H), 7.90-7.84 (m, 2H), 7.72 (d, J = 8.1 Hz, 1H), 7.61 (dd, J = 8.8, 2.4 Hz, 1H), 7.39-7.26 (m, 1H), 6.88 (s, 1H), 6.51-6.30 (m, 2H), 5.77 (dd, J = 9.8, 1.9 Hz, 1H).

### Example 9: Preparation of N-(4-((3-chloro-5-(trifluoromethyl)pyridin-2-yl)amino)-3-(1-methyl-1H-imidazol-4-yl)phenyl)acrylamide

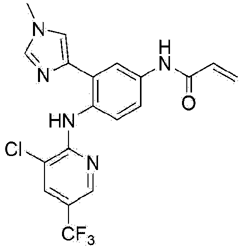

The title compound (15 mg, yield: 12%) was obtained in the same manner as in Example 4, except that 1-methyl-4-(tributylstannyl)-1H-imidazole was used instead of 2-(tributylstannyl)pyridine in step 1 of Example 4, and 3-chloro-4-(trifluoromethyl)pyridin-2-amine was used instead of 5-(trifluoromethyl)pyridin-2-amine in step 2 of Example 4.

¹H NMR (500 MHz, CDCl₃) δ 11.83 (s, 1H), 8.65 (d, J = 8.9 Hz, 1H), 8.36 (s, 1H), 8.24 (s, 1H), 7.75 (s, 1H), 7.55 (s, 1H), 7.48 (s, 1H), 7.26 (s, 1H), 7.18 (d, J = 8.0 Hz, 1H), 6.47 (d, J = 16.7 Hz, 1H), 6.30 (dd, J = 16.7, 10.2 Hz, 1H), 5.79 (d, J = 10.1 Hz, 1H), 3.75 (s, 3H).

### Example 10: Preparation of N-(4-((4,5-dichloropyridin-2-yl)amino)-3-(1-methyl-1H-imidazol-4-yl)phenyl)acrylamide

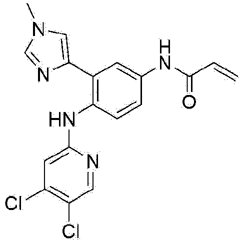

The title compound (17 mg, yield: 45%) was obtained in the same manner as in Example 4, except that 1-methyl-4-(tributylstannyl)-1H-imidazole was used instead of 2-(tributylstannyl)pyridine in step 1 of Example 4, and 4,5-dichloro-(trifluoromethyl)pyridin-2-amine was used instead of 5-(trifluoromethyl)pyridin-2-amine in step 2 of Example 4.

¹H NMR (500 MHz, DMSO) δ 10.86 (s, 1H), 10.15 (s, 1H), 8.26 (s, 1H), 8.09 (d, J = 2.3 Hz, 1H), 7.98 (d, J = 8.8 Hz, 1H), 7.82 (s, 1H), 7.49-7.45 (m, 2H), 6.99 (s, 1H), 6.44 (dd, J = 16.9, 10.1 Hz, 1H), 6.25 (dd, J = 17.0, 1.8 Hz, 1H), 5.74 (dd, J = 10.3, 1.6 Hz, 1H), 3.72 (s, 3H).

### Example 11: Preparation of N-(6-((4-fluorobenzyl)amino)-[1,1'-biphenyl]-3-yl)acrylamide

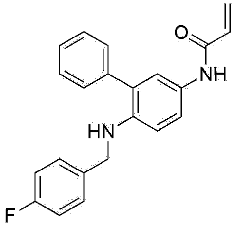

The title compound (16 mg, yield: 47%) was obtained in the same manner as in Example 1, except that 4-fluorobenzylamine was used instead of cyclohexanamine in step 1 of Example 1.

¹H NMR (500 MHz, CDCl₃) δ 7.50-7.29 (m, 8H), 7.28-7.23 (m, 2H), 7.01-6.97 (m, 2H), 6.56 (d, J = 8.7 Hz, 1H), 6.37 (d, J = 16.5 Hz, 1H), 6.21 (dd, J = 16.8, 10.2 Hz, 1H), 5.68 (dd, J = 10.2, 0.9 Hz, 1H), 4.35-4.25 (m, 3H).

### Example 12: Preparation of N-(4-((4-fluorobenzyl)amino)-3-(pyridin-2-yl)phenyl)acrylamide

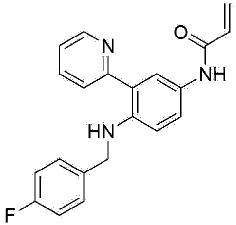

The title compound (18 mg, yield: 53%) was obtained in the same manner as in Example 1, except that 4-fluorobenzylamine was used instead of cyclohexanamine in step 1 of Example 1, and 2-tributylstannylpyridine was used instead of phenylboronic acid in step 2 of Example 1.

¹H NMR (500 MHz, CDCl₃) δ 8.84 (brs, 1H) 8.53 (d, J = 4.7 Hz, 1H), 8.04 (d, J = 2.3 Hz, 1H), 7.72-7.71 (m, 2H), 7.54 (brs, 1H), 7.33-7.30 (m, 2H), 7.20 (dd, J = 8.8, 2.4 Hz, 1H), 7.15 (dd, J = 8.6, 4.5 Hz, 1H), 7.01-6.97 (m, 2H), 6.57 (d, J = 8.8 Hz, 1H), 6.40 (ddd, J = 16.8, 7.3, 1.0 Hz, 1H), 6.24 (dd, J = 16.8, 10.2 Hz, 1H), 5.69 (dd, J = 10.3, 0.8 Hz, 1H), 4.41 (s, 2H).

### Example 13: Preparation of N-(3-(5-chloropyridin-2-yl)-4-((5-(trifluoromethyl)pyridin-2-yl)amino)phenyl)acrylamide

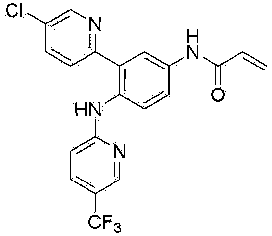

The title compound (10 mg, yield: 25%) was obtained in the same manner as in Example 4, except that 5-chloro-2-(tributylstannyl)pyridine was used instead of 2-(tributylstannyl)pyridine in step 1 of Example 4.

¹H NMR (500 MHz, CDCl₃) δ 10.93 (s, 1H), 8.61 (d, J = 2.1 Hz, 1H), 8.42 (s, 1H), 8.24-8.19 (m, 2H), 7.80-7.56 (m, 4H), 7.31 (dd, J = 8.8, 2.2 Hz, 1H), 6.78 (d, J = 8.8 Hz, 1H), 6.46 (d, J = 16.4 Hz, 1H), 6.28 (dd, J = 16.8, 10.2 Hz, 1H), 5.78 (d, J = 10.9 Hz, 1H).

### Example 14: Preparation of N-(4-(pyridin-2-yl)-3-((4-(trifluoromethyl)phenyl)amino)phenyl)acrylamide

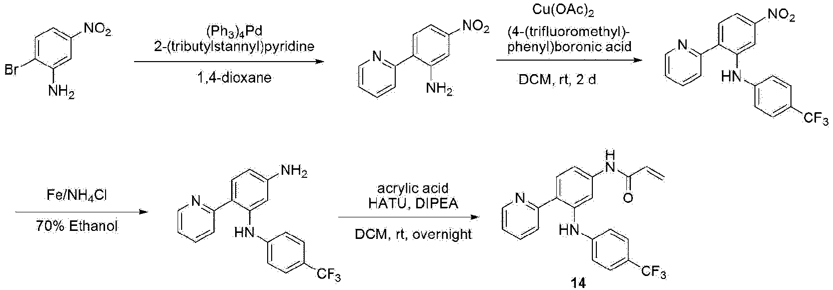

### (Step 1)

In a sealed tube, 2-bromo-5-nitroaniline (1.0 mmol, 0.21 g, 1.0 eq) was dissolved in 1,4-dioxane (20 mL), and then 2-(tributylstannyl)pyridine (1.2 mmol, 0.44 g, 1.2 eq) and (Ph₃P)₄Pd (0.1 mmol, 0.11 g, 0.1 eq) were sequentially added thereto and allowed to react at 150°C overnight. After the reaction was completed, the solvent was removed, and the resulting material was isolated and purified by column chromatography (ethyl acetate:hexane=1:3(v:v)) to give 5-nitro-2-(pyridin-2-yl)aniline (0.15 g, yield: 70%).

### (Step 2)

5-Nitro-2-(pyridin-2-yl)aniline (0.69 mmol, 0.15 g, 1.0 eq), Cu(OAc)₂ (1.72 mmol, 0.31 g, 2.5 eq), (4-(trifluoromethyl)phenyl)boronic acid (1.4 mmol, 0.27 g, 2.0 eq), and dichloromethane (10 mL) was sequentially added to a flask, and then stirred. Pyridine (2.76 mmol, 0.22 g, 4.0 eq) was added thereto, and then stirred at room temperature for 48 hours. After the reaction was completed, Cu(OAc)₂ was removed, and the resulting material was isolated and purified by column chromatography (ethyl acetate/hexane=1:3(v:v)) to give 5-nitro-2-(pyridin-2-yl)-N-(4-(trifluoromethyl)phenyl)aniline (0.22 g, yield: 88%).

### (Step 3)

5-Nitro-2-(pyridin-2-yl)-N-(4-(trifluoromethyl)phenyl)aniline (0.61 mmol, 0.22 g, 1.0 eq), Fe (6 mmol, 0.33 g, 10 eq), NH₄Cl (0.61 mmol, 0.032 g, 1.0 eq) and 70% ethanol solution (10 mL) were added to a flask, and then stirred at room temperature for 12 hours. After the reaction was completed, Fe and NH₄Cl were removed to give 6-(pyridin-2-yl)-N¹-(4-(trifluoromethyl)phenyl)benzene-1,3-diamine (0.2 g, yield: 99%).

### (Step 4)

6-(Pyridin-2-yl)-N¹-(4-(trifluoromethyl)phenyl)benzene-1,3-diamine (0.1 mmol, 0.033 g, 1.0 eq), acrylic acid (0.1 mmol, 0.0072 g, 1.0 eq), 1-[bis(dimethylamino)methylene]-1H-1,2,3-triazolo[4,5-b]pyridinium 3-oxide hexafluorophosphate (0.12 mmol, 0.0046 g), and N,N-diisopropylethylamine (0.1 mL) were dissolved in dichloromethane (1 mL), and allowed to react overnight at room temperature. After the reaction was completed, dichloromethane was removed, and the resulting material was purified by column chromatography to give Compound 14 (13 mg, yield: 33%).

¹H NMR (500 MHz, CDCl₃) δ 10.85 (s, 1H), 8.62 (d, J = 4.2 Hz, 1H), 7.83-7.75 (m, 2H), 7.70 (d, J = 8.1 Hz, 1H), 7.63 (d, J = 8.5 Hz, 1H), 7.50 (d, J = 8.5 Hz, 2H), 7.45 (s, 1H), 7.31-7.24 (m, 3H), 7.22 (dd, J = 6.8, 5.2 Hz, 1H), 6.45 (dd, J = 16.8, 0.4 Hz, 1H), 6.25 (dd, J = 16.8, 10.3 Hz, 1H), 5.78 (d, J = 10.8 Hz, 1H).

### Example 15: Preparation of N-(3-(pyrimidin-4-yl)-4-((5-(trifluoromethyl)pyridin-2-yl)amino)phenyl)acrylamide

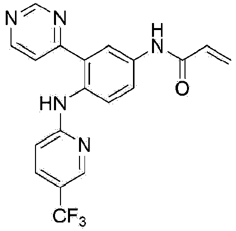

The title compound (5 mg, yield: 12%) was obtained in the same manner as in Example 4, except that 4-(tributylstannyl)pyrimidine was used instead of 2-(tributylstannyl)pyridine in step 1 of Example 4.

¹H NMR (500 MHz, CDCl₃) δ 11.48 (s, 1H), 9.27 (s, 1H), 8.78 (d, J = 5.4 Hz, 1H), 8.53-8.29 (m, 3H), 7.84-7.61 (m, 3H), 7.35 (dd, J = 8.9, 2.4 Hz, 1H), 6.84 (d, J = 8.7 Hz, 1H), 6.47 (d, J = 17.2 Hz, 1H), 6.29 (dd, J = 16.8, 10.2 Hz, 1H), 5.80 (d, J = 10.3 Hz, 1H).

### Example 16: Preparation of N-(4-((4,5-dichloropyridin-2-yl)amino)-3-(pyrimidin-4-yl)phenyl)acrylamide

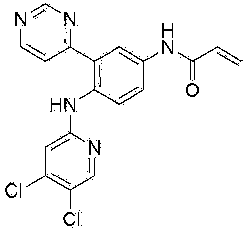

The title compound (7 mg, yield: 19%) was obtained in the same manner as in Example 4, except that 4-(tributylstannyl)pyrimidine was used instead of 2-(tributylstannyl)pyridine in step 1 of Example 4, and 4,5-dichloro-(trifluoromethyl)pyridin-2-amine was used instead of 5-(trifluoromethyl)pyridin-2-amine in step 2 of Example 4.

¹H NMR (500 MHz, CDCl₃) δ 11.33 (s, 1H), 9.27 (s, 1H), 8.78 (d, J = 5.5 Hz, 1H), 8.44 (d, J = 1.5 Hz, 1H), 8.27-8.14 (m, 2H), 7.77-7.72 (m, 2H), 7.33 (dd, J = 8.9, 2.3 Hz, 1H), 6.93 (s, 1H), 6.46 (d, J = 16.8 Hz, 1H), 6.29 (dd, J = 16.8, 10.2 Hz, 1H), 5.79 (d, J = 10.3 Hz, 1H).

### Example 17: Preparation of N-(3-(pyridin-4-yl)-4-((5-(trifluoromethyl)pyridin-2-yl)amino)phenyl)acrylamide

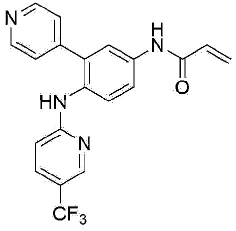

The title compound (16 mg, yield: 42%) was obtained in the same manner as in Example 4, except that 4-(tributylstannyl)pyridine was used instead of 2-(tributylstannyl)pyridine in step 1 of Example 4.

¹H NMR (500 MHz, CDCl₃) δ 8.65-8.64 (m, 2H), 8.39 (s, 1H), 7.75 (s, 1H), 7.69-7.59 (m, 3H), 7.41-7.30 (m, 3H), 6.66 (d, J = 8.7 Hz, 1H), 6.52 - 6.39 (m, 2H), 6.26 (dd, J = 16.8, 10.3 Hz, 1H), 5.82 (d, J = 10.3 Hz, 1H).

### Example 18: Preparation of N-(4-((4,5-dichloropyridin-2-yl)amino)-3-(pyridin-4-yl)phenyl)acrylamide

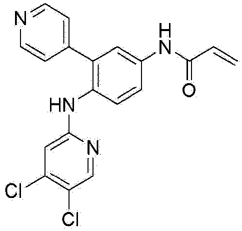

The title compound (14 mg, yield: 36%) was obtained in the same manner as in Example 4, except that 4-(tributylstannyl)pyridine was used instead of 2-(tributylstannyl)pyridine in step 1 of Example 4, and 4,5-dichloro-(trifluoromethyl)pyridin-2-amine was used instead of 5-(trifluoromethyl)pyridin-2-amine in step 2 of Example 4.

¹H NMR (500 MHz, CDCl₃) δ 8.63-8.62 (m, 2H), 8.11 (s, 1H), 7.74 (s, 1H), 7.65-7.55 (m, 3H), 7.32-7.31 (d, J = 5.9 Hz, 2H), 6.72 (s, 1H), 6.46 (dd, J = 16.8, 0.7 Hz, 1H), 6.34-6.22 (m, 2H), 5.80 (dd, J = 10.2, 0.8 Hz, 1H).

### Example 19: Preparation of N-(4-(5-chloro-4-fluoro-1H-indol-1-yl)-3-(pyridin-2-yl)phenyl)acrylamide

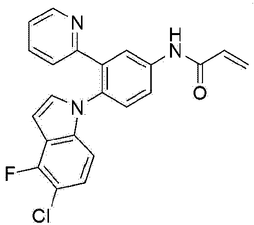

The title compound (19 mg, yield: 48%) was obtained in the same manner as in Example 4, except that 5-chloro-4-fluoro-1H-indole was used instead of 5-(trifluoromethyl)pyridin-2-amine in step 2 of Example 4.

¹H NMR (500 MHz, CDCl₃) δ 8.58 (d, J = 4.4 Hz, 1H), 8.16-8.06 (m, 2H), 7.90 (d, J = 2.4 Hz, 1H), 7.43 (d, J = 8.6 Hz, 1H), 7.30 (td, J = 7.8, 1.7 Hz, 1H), 7.10 (dd, J = 6.7, 5.0 Hz, 1H), 7.01 (dd, J = 8.6, 7.0 Hz, 1H), 6.95 (d, J = 3.2 Hz, 1H), 6.81 (d, J = 8.7 Hz, 1H), 6.60 (d, J = 3.1 Hz, 1H), 6.50 (dd, J = 14.8, 12.5 Hz, 1H), 6.29 (dd, J = 16.9, 10.3 Hz, 1H), 5.88-5.77 (m, 1H), 5.30 (s, 1H).

### Example 20: Preparation of N-(4-((5-cyclopropylpyridin-2-yl)amino)-3-(pyridin-2-yl)phenyl)acrylamide

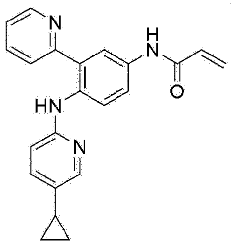

The title compound (3 mg, yield: 9%) was obtained in the same manner as in Example 4, except that 5-cyclopropylpyridin-1-amine was used instead of 5-(trifluoromethyl)pyridin-2-amine in step 2 of Example 4.

¹H NMR (500 MHz, CDCl₃) δ 10.82 (s, 1H), 8.65 (d, J = 4.6 Hz, 1H), 8.23-8.00 (m, 3H), 7.81-7.65 (m, 2H), 7.46 (s, 1H), 7.31 (dd, J = 8.8, 2.0 Hz, 1H), 7.24-7.19 (m, 1H), 7.16 (dd, J = 8.5, 2.2 Hz, 1H), 6.77 (d, J = 8.5 Hz, 1H), 6.44 (d, J = 17.1 Hz, 1H), 6.26 (dd, J = 16.8, 10.2 Hz, 1H), 5.75 (d, J = 10.2 Hz, 1H), 1.82-1.75 (m, 1H), 0.93-0.86 (m, 2H), 0.62-0.55 (m, 2H).

### Example 21: Preparation of N-(4-((3-fluoro-5-(trifluoromethyl)pyridin-2-yl)amino)-3-(pyridin-2-yl)phenyl)acrylamide

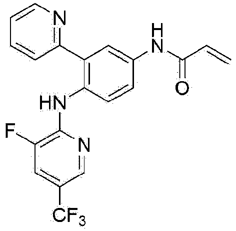

The title compound (9 mg, yield: 22%) was obtained in the same manner as in Example 4, except that 3-fluoro-5-(trifluoromethyl)pyridin-2-amine was used instead of 5-(trifluoromethyl)pyridin-2-amine in step 2 of Example 4.

¹H NMR (500 MHz, CDCl₃) δ 12.56 (s, 1H), 8.76 (d, J = 8.9 Hz, 1H), 8.68 (d, J = 4.7 Hz, 1H), 8.43 (s, 1H), 8.26 (s, 1H), 7.92-7.75 (m, 2H), 7.45-7.35 (m, 2H), 7.35-7.26 (m, 2H), 6.47 (d, J = 16.8 Hz, 1H), 6.28 (dd, J = 16.8, 10.2 Hz, 1H), 5.80 (d, J = 10.2 Hz, 1H).

### Example 22: Preparation of N-(6-(((1r,4r)-4-hydroxycyclohexyl)amino)-5-phenylpyridin-3-yl)acrylamide

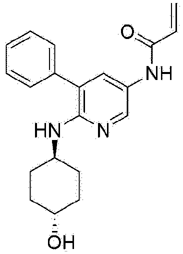

The title compound (11 mg, yield: 34%) was obtained in the same manner as in Example 1, except that trans-4-aminocyclohexan-1-ol and 3-bromo-2-chloro-5-nitropyridine were respectively used instead of cyclohexanamine and 2-bromo-1-fluoro-4-nitrobenzene in step 1 of Example 1.

¹H NMR (500 MHz, CDCl₃) δ 8.12 (d, J = 2.2 Hz, 1H), 7.84 (s, 1H), 7.76 (d, J = 2.2 Hz, 1H), 7.46-7.31 (m, 6H), 6.43-6.35 (m, 1H), 6.27 (dd, J = 16.9, 10.2 Hz, 1H), 5.73 (d, J = 12.2 Hz, 1H), 4.41-4.33 (m, 1H), 3.94-3.83 (m, 1H), 3.63-3.53 (m, 1H), 2.12-2.06 (m, 2H), 1.98-1.90 (m, 2H), 1.50-1.40 (m, 2H), 1.15-1.04 (m, 2H).

### Example 23: Preparation of (E)-N-(3-(4-fluorostyryl)-4-((2-(thiazol-2-yl)ethyl)amino)phenyl)acrylamide

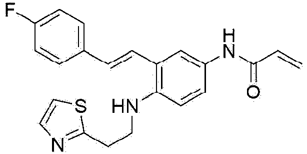

The title compound (14 mg, yield: 36%) was obtained in the same manner as in Example 1, except that 2-(thiazol-2-yl)ethane-1-amine was used instead of cyclohexanamine in step 1 of Example 1, and (E)-(4-fluorostyryl)boronic acid was used instead of phenylboronic acid in step 2 of Example 1.

¹H NMR (500 MHz, CDCl₃) δ 7.72-7.59 (m, 3H), 7.43-7.40 (m, 2H), 7.35-7.30 (m, 1H), 7.21 (d, J = 3.3 Hz, 1H), 7.08-6.97 (m, 3H), 6.87 (d, J = 16.0 Hz, 1H), 6.64 (d, J = 8.7 Hz, 1H), 6.40 (d, J = 16.8 Hz, 1H), 6.25 (dd, J = 16.8, 10.2 Hz, 1H), 5.69 (d, J = 10.1 Hz, 1H), 4.53 (brs, 1H), 3.56 (t, J = 6.3 Hz, 2H), 3.33 (t, J = 6.3 Hz, 2H).

### Example 24: Preparation of (E)-N-(3-(4-fluorostyryl)-4-((2-(thiazol-2-yl)ethyl)amino)phenyl)ethanesulfonamide

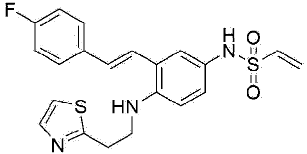

The title compound (8 mg, yield: 18%) was obtained in the same manner as in Example 1, except that 2-(thiazol-2-yl)ethane-1-amine was used instead of cyclohexanamine in step 1 of Example 1, (E)-(4-fluorostyryl)boronic acid was used instead of phenylboronic acid in step 2 of Example 1, and N-(3-dimethylaminopropyl)-N-ethylcarbodiimide hydrochloride was not used and ethenesulfonyl chloride was used instead of acrylic acid in step 4 of Example 1.

¹H NMR (500 MHz, CDCl₃) δ 7.67 (d, J = 3.2 Hz, 1H), 7.48-7.45 (m, 2H), 7.25-7.21 (m, 2H), 7.08-7.05 (m, 3H), 6.98 (d, J = 15.9 Hz, 1H), 6.87 (d, J = 16.0 Hz, 1H), 6.63 (d, J = 8.6 Hz, 1H), 6.59-6.53 (m, 1H), 6.18 (d, J = 16.6 Hz, 1H), 6.51 (brs, 1H), 5.90 (d, J = 10.0 Hz, 1H), 4.71 (brs, 1H), 3.60-3.53 (m, 2H), 3.36 (t, J = 6.2 Hz, 2H).

### Example 25: Preparation of N-(4-(cyclohexylamino)-3-(pyridin-3-yl)phenyl)acrylamide

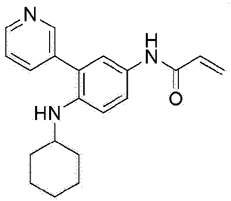

The title compound (7 mg, yield: 22%) was obtained in the same manner as in Example 2, except that 3-(tributylstannyl)pyridine was used instead of 2-(tributylstannyl)pyridine in step 1 of Example 2.

¹H NMR (500 MHz, CDCl₃) δ 8.62 (d, J = 1.2 Hz, 1H), 8.56 (d, J = 4.0 Hz, 1H), 8.26 (s, 1H), 7.72 (d, J = 7.8 Hz, 1H), 7.48 (dd, J = 8.8, 2.1 Hz, 1H), 7.37-7.31 (m, 1H), 7.27 (d, J = 2.2 Hz, 1H), 6.68-6.64 (m, 2H), 6.36 (d, J = 16.1 Hz, 1H), 6.27 (dd, J = 16.9, 10.0 Hz, 1H), 5.68 - 5.57 (m, 1H), 3.25-3.21 (m, 1H), 1.95-1.88 (m, 2H), 1.70-1.50 (m, 2H), 1.40-0.95 (m, 6H).

### Example 26: Preparation of N-(4-(cyclopentylamino)-3-(pyridin-2-yl)phenyl)acrylamide

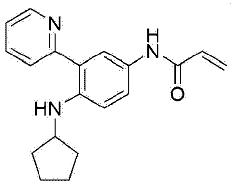

The title compound (4 mg, yield: 14%) was obtained in the same manner as in Example 1, except that cyclopentanamine was used instead of cyclohexanamine in step 1 of Example 1, and 2-tributylstannylpyridine was used instead of phenylboronic acid in step 2 of Example 1.

¹H NMR (500 MHz, CDCl₃) δ 8.07 (s, 1H), 8.02 (s, 1H), 7.48-7.41 (m, 2H), 7.10-7.02 (m, 3H), 6.96 (d, J = 15.9 Hz, 1H), 6.77 (d, J = 15.9 Hz, 1H), 6.43 (d, J = 16.9 Hz, 1H), 6.26 (dd, J = 16.8, 10.3 Hz, 1H), 5.77 (d, J = 10.2 Hz, 1H), 3.63-3.62 (m, 1H), 2.25-2.12 (m, 2H), 2.05-1.96 (m, 2H), 1.52 - 1.46 (m, 2H), 1.25-1.20 (m, 2H).

### Example 27: Preparation of (E)-N-(5-(4-fluorostyryl)-6-((2-(thiazol-2-yl)ethyl)amino)pyridin-3-yl)acrylamide

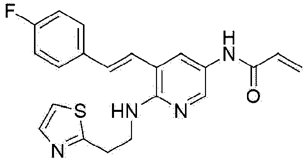

The title compound (19 mg, yield: 45%) was obtained in the same manner as in Example 1, except that 2-(thiazol-2-yl)ethane-1-amine and 3-bromo-2-chloro-5-nitropyridine were respectively used instead of cyclohexanamine and 2-bromo-1-fluoro-4-nitrobenzene in step 1 of Example 1, and (E)-(4-fluorostyryl)boronic acid was used instead of phenylboronic acid in step 2 of Example 1.

¹H NMR (500 MHz, CDCl₃) δ 8.07 (s, 1H), 8.02 (s, 1H), 7.67 (d, J = 3.1 Hz, 1H), 7.46-7.39 (m, 2H), 7.21 (d, J = 3.1 Hz, 1H), 7.05-7.02 (m, 2H), 6.92 (d, J = 16.1 Hz, 1H), 6.83 (d, J = 16.0 Hz, 1H), 6.42 (d, J = 16.9 Hz, 1H), 6.27 (dd, J = 16.8, 10.2 Hz, 1H), 5.74 (d, J = 10.0 Hz, 1H), 5.39 (s, 1H), 4.56 (s, 1H), 3.85 (d, J = 5.7 Hz, 2H), 3.35 (t, J = 6.2 Hz, 2H).

### Example 28: Preparation of (E)-N-(5-(4-fluorostyryl)-6-((2-(thiazol-2-yl)ethyl)amino)pyridin-3-yl)ethanesulfonamide

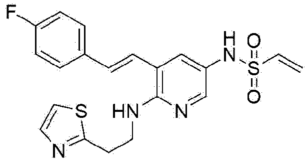

The title compound (4 mg, yield: 11%) was obtained in the same manner as in Example 1, except that 2-(thiazol-2-yl)ethane-1-amine and 3-bromo-2-chloro-5-nitropyridine were respectively used instead of cyclohexanamine and 2-bromo-1-fluoro-4-nitrobenzene in step 1 of Example 1, (E)-(4-fluorostyryl)boronic acid was used instead of phenylboronic acid in step 2 of Example 1, and N-(3-dimethylaminopropyl)-N-ethylcarbodiimide hydrochloride was not used and ethenesulfonyl chloride was used instead of acrylic acid in step 4 of Example 1.

¹H NMR (500 MHz, CDCl₃) δ 7.89 (d, J = 2.2 Hz, 1H), 7.67 (d, J = 3.3 Hz, 1H), 7.51 (d, J = 2.1 Hz, 1H), 7.48-7.45 (m, 2H), 7.22 (d, J = 3.2 Hz, 1H), 7.08-7.06 (m, 2H), 6.92 (d, J = 16.1 Hz, 1H), 6.82 (d, J = 16.0 Hz, 1H), 6.57 (dd, J = 16.5, 9.9 Hz, 1H), 6.18 (d, J = 16.6 Hz, 1H), 5.94 (d, J = 9.9 Hz, 1H), 5.59 (t, J = 5.2 Hz, 1H), 3.89-3.86 (m, 2H), 3.40-3.29 (m, 3H).

### Example 29: Preparation of N-(3-fluoro-5-(pyridin-2-yl)-4-((5-(trifluoromethyl)pyridin-2-yl)amino)phenyl)acrylamide

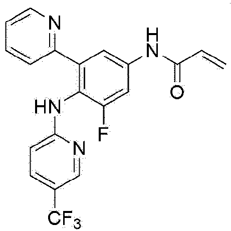

The title compound (10 mg, yield: 25%) was obtained in the same manner as in Example 4, except that 1-bromo-2,3-difluoro-5-nitrobenzene was used instead of 2-bromo-1-fluoro-4-nitrobenzene in step 1 of Example 4.

¹H NMR (500 MHz, CDCl₃) δ 9.51 (s, 1H), 8.72 (d, J = 4.4 Hz, 1H), 8.40 (s, 1H), 7.87-7.74 (m, 2H), 7.69 (d, J = 8.0 Hz, 1H), 7.65-7.56 (m, 2H), 7.38-7.30 (m, 2H), 6.60 - 6.48 (m, 2H), 6.29 (dd, J = 16.8, 10.2 Hz, 1H), 5.87 (d, J = 10.6 Hz, 1H).

### Example 30: Preparation of N-(4-((5-chloro-4-cyclopropylpyridin-2-yl)amino)-3-(pyridin-2-yl)phenyl)acrylamide

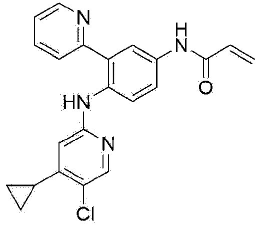

The title compound (12 mg, yield: 30%) was obtained in the same manner as in Example 4, except that 5-chloro-4-cyclopropylpyridin-2-amine was used instead of 5-(trifluoromethyl)pyridin-2-amine in step 2 of Example 4.

¹H NMR (500 MHz, CDCl₃) δ 10.90 (s, 1H), 8.67 (d, J = 4.4 Hz, 1H), 8.20 (d, J = 2.1 Hz, 1H), 8.09 (s, 1H), 8.06 (d, J = 8.9 Hz, 1H), 7.77 (dd, J = 11.9, 4.5 Hz, 2H), 7.73 (d, J = 7.9 Hz, 1H), 7.34 (dd, J = 8.8, 2.2 Hz, 1H), 7.27-7.21 (m, 1H), 6.45 (d, J = 16.7 Hz, 1H), 6.35-6.23 (m, 2H), 5.80-5.71 (m, 1H), 2.19-2.13 (m, 1H), 1.16-1.02 (m, 2H), 0.81-0.66 (m, 2H).

### Example 31: Preparation of N-(3-(1-methyl-1H-imidazol-4-yl)-4-((4-(trifluoromethyl)pyridin-2-yl)amino)phenyl)acrylamide

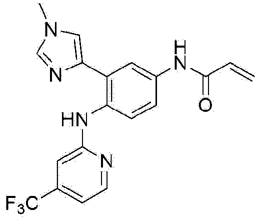

The title compound (3 mg, yield: 9%) was obtained in the same manner as in Example 4, except that 1-methyl-4-(tributylstannyl)-1H-imidazole was used instead of 2-(tributylstannyl)pyridine in step 1 of Example 4, and 4-(trifluoromethyl)pyridin-2-amine was used instead of 5-(trifluoromethyl)pyridin-2-amine in step 2 of Example 4.

¹H NMR (500 MHz, CDCl₃) δ 11.34 (s, 1H), 8.44 (d, J = 8.8 Hz, 1H), 8.35 (d, J = 5.1 Hz, 1H), 8.22 (s, 1H), 7.54 (s, 1H), 7.48 (s, 1H), 7.26 (s, 1H), 7.19 (d, J = 7.2 Hz, 1H), 7.01 (s, 1H), 6.86 (d, J = 4.9 Hz, 1H), 6.47 (d, J = 16.8 Hz, 1H), 6.29 (dd, J = 16.8, 10.2 Hz, 1H), 5.78 (d, J = 10.2 Hz, 1H), 3.74 (s, 3H).

### Example 32: Preparation of (E)-4-(dimethylamino)-N-(3-(1-methyl-1H-pyrazol-3-yl)-4-((4-(trifluoromethyl)pyridin-2-yl)amino)phenyl)-2-butenamide

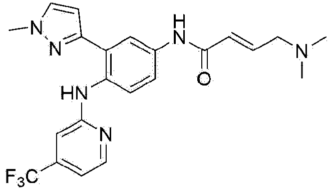

The title compound (23 mg, yield: 52%) was obtained in the same manner as in Example 4, except that 1-methyl-4-(tributylstannyl)-1H-pyrazole was used instead of 2-(tributylstannyl)pyridine in step 1 of Example 4, 4-(trifluoromethyl)pyridin-2-amine was used instead of 5-(trifluoromethyl)pyridin-2-amine in step 2 of Example 4, and (E)-4-(dimethylamino)2-butenoyl chloride HCl salt was used instead of acrylic acid and HATU was not used in step 4 of Example 4.

¹H NMR (500 MHz, DMSO-*d*₆) δ 10.41 (s, 1H), 10.31 (brs, 1H), 10.18 (s, 1H), 8.35 (d, J = 5.2 Hz, 1H), 8.15 (d, J = 8.9 Hz, 1H), 8.09 (d, J = 2.3 Hz, 1H), 7.82 (d, J = 2.2 Hz, 1H), 7.63 (dd, J = 8.9, 2.3 Hz, 1H), 7.06 (s, 1H), 7.00 (d, J = 5.2 Hz, 1H), 6.81 - 6.74 (m, 1H), 6.55 (d, J = 2.2 Hz, 1H), 6.46 (d, J = 15.3 Hz, 1H), 3.97 (s, 3H), 3.89 (brs, 2H), 2.74 (s, 6H).

### Example 33: Preparation of N-(4-((5-isopropylpyridin-2-yl)amino)-3-(pyridin-2-yl)phenyl)acrylamide

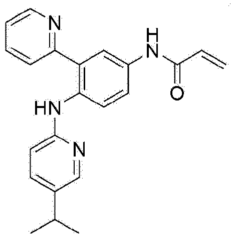

The title compound (3 mg, yield: 8%) was obtained in the same manner as in Example 4, except that 5-isopropylpyridin-2-amine was used instead of 5-(trifluoromethyl)pyridin-2-amine in step 2 of Example 4.

¹H NMR (500 MHz, CDCl₃) δ 10.82 (brs, 1H), 8.66 (d, J = 4.3 Hz, 1H), 8.17 (s, 1H), 8.12-8.04 (m, 2H), 7.79-7.72 (m, 2H), 7.43-7.32 (m, 3H), 7.23 (t, J = 5.6 Hz, 1H), 6.82 (d, J = 8.5 Hz, 1H), 6.45 (d, J = 16.8 Hz, 1H), 6.26 (dd, J = 16.8, 10.2 Hz, 1H), 5.76 (d, J = 10.2 Hz, 1H), 2.83 (dt, J = 13.8, 6.8 Hz, 1H), 1.22 (d, J = 6.9 Hz, 1H).

### Example 34: Preparation of N-(4-((5-ethynylpyridin-2-yl)amino)-3-(1-methyl-1H-pyrazol-3-yl)phenyl)acrylamide

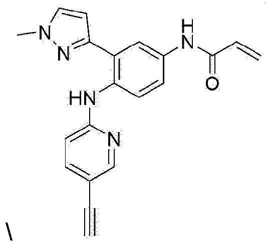

The title compound (22 mg, yield: 58%) was obtained in the same manner as in Example 4, except that 1-methyl-4-(tributylstannyl)-1H-pyrazole was used instead of 2-(tributylstannyl)pyridine in step 1 of Example 4, and 5-ethynylpyridin-2-amine was used instead of 5-(trifluoromethyl)pyridin-2-amine in step 2 of Example 4.

¹H NMR (500 MHz, CDCl₃) δ 10.48 (s, 1H), 8.38 (dd, J = 5.2, 3.3 Hz, 2H), 8.13 (d, J = 2.0 Hz, 1H), 7.55 (dd, J = 8.6, 1.9 Hz, 1H), 7.41 (d, J = 2.0 Hz, 1H), 7.32 (dd, J = 8.8, 1.9 Hz, 1H), 7.22 (s, 1H), 6.77 (d, J = 8.6 Hz, 1H), 6.64 (d, J = 1.8 Hz, 1H), 6.45 (d, J = 16.8 Hz, 1H), 6.26 (dd, J = 16.8, 10.2 Hz, 1H), 5.78 (d, J = 10.2 Hz, 1H), 4.00 (s, 3H), 3.10 (s, 1H).

### Example 35: Preparation of N-(3-(1-methyl-1H-imidazol-4-yl)-4-((4-(trifluoromethyl)phenyl)amino)phenyl)acrylamide

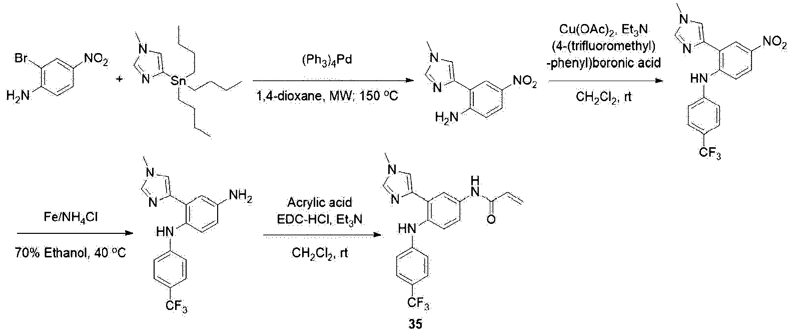

### (Step 1)

In a sealed tube, 2-bromo-4-nitroaniline (4.6 mmol, 1.0 g, 1.0 eq) was dissolved in 1,4-dioxane (9 mL), and then 1-methyl-4-(tributylstannyl)-1H-imidazole (5.5 mmol, 2.1 g, 1.2 eq), and (Ph₃P)₄Pd (0.46 mmol, 0.53 g, 0.1 eq) were added sequentially thereto and allowed to react at 150°C for 2 hours. After the reaction was completed, the solvent was removed, and the resulting material was isolated and purified by column chromatography (ethyl acetate:hexane = 1:1 (v:v)) to give 2-(1-methyl-1H-imidazol-4-yl)-4-nitroaniline (0.64 g, yield: 64%).

### (Step 2)

2-(1-Methyl-1H-imidazol-4-yl)-4-nitroaniline (5.1 mmol, 1.1 g, 1.0 eq), Cu(OAc)₂ (6.1 mmol, 1.1 g, 1.2 eq), (4-(trifluoromethyl)phenyl)boronic acid (6.1 mmol, 1.2 g, 1.2 eq), and dichloromethane (25 mL) were sequentially added to a flask, and then stirred. Triethylamine (10 mmol, 1.0 g, 2.0 eq) was added thereto and then stirred at room temperature for 2 hours. After the reaction was completed, Cu(OAc)₂ was removed, and the resulting material was purified by column chromatography (ethyl acetate/hexane = 1:3 (v:v)) to give 2-(1-methyl-1H-imidazol-4-yl)-4-nitro-N-(4-(trifluoromethyl)phenyl)aniline (1.2 g, yield: 67%).

### (Step 3)

2-(1-Methyl-1H-imidazol-4-yl)-4-nitro-N-(4-(trifluoromethyl)phenyl)aniline (3.1 mmol, 1.1 g, 1.0 eq), Fe (31 mmol, 1.7 g, 10 eq), NH₄Cl (3.1 mmol, 0.17 g, 1.0 eq) and 70% ethanol solution (15 mL) were added to a flask, and then stirred at 40°C for 4 hours. After the reaction was reacted, Fe and NH₄Cl were removed, and the resulting material was isolated and purified by column chromatography (ethyl acetate) to give 2-(1-methyl-1H-imidazol-4-yl)-N¹-(4-(trifluoromethyl)phenyl)benzene-1,4-diamine (0.60 g, yield: 59%).

### (Step 4)

2-(1-Methyl-1H-imidazol-4-yl)-N¹-(4-(trifluoromethyl)phenyl)benzene-1,4-diamine (1.8 mmol, 0.60 g, 1.0 eq), acrylic acid (2.2 mmol, 0.16 g, 1.2 eq), N-(3-dimethylaminopropyl)-N-ethylcarbodiimide hydrochloride (2.7 mmol, 0.52 g, 1.5 eq), and triethylamine (0.76 mL) were dissolved in dichloromethane (9 mL), and allowed to react at room temperature for 3 hours. After the reaction was reacted, dichloromethane was removed, and the resulting material was isolated and purified by column chromatography (ethyl acetate) to give the title compound (0.35 g, yield: 51%).

¹H NMR (500 MHz, DMSO-*d*₆) δ 10.18 (s, 1H), 9.38 (s, 1H), 8.15 (d, J = 2.3 Hz, 1H), 7.75 (s, 1H), 7.57 (dd, J = 8.7, 2.3 Hz, 1H), 7.47 (d, J = 8.6 Hz, 2H), 7.42 (s, 1H), 7.31 (d, J = 8.7 Hz, 1H), 6.98 (d, J = 8.5 Hz, 2H), 6.45 (dd, J = 17.0, 10.1 Hz, 1H), 6.26 (dd, J = 17.0, 1.8 Hz, 1H), 5.75 (dd, J = 10.2, 1.8 Hz, 1H), 3.67 (s, 3H).

### Example 36: Preparation of N-(4-((3-fluoro-5-(trifluoromethyl)pyridin-2-yl)amino)-3-(1-methyl-1H-imidazol-4-yl)phenyl)acrylamide

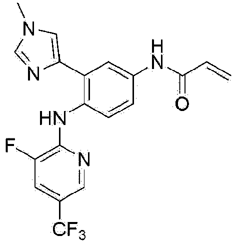

The title compound (5 mg, yield: 12%) was obtained in the same manner as in Example 4, except that 1-methyl-4-(tributylstannyl)-1H-imidazole was used instead of 2-(tributylstannyl)pyridine in step 1 of Example 4, and 3-fluoro-5-(trifluoromethyl)pyridin-2-amine was used instead of 5-(trifluoromethyl)pyridin-2-amine in step 2 of Example 4.

¹H NMR (500 MHz, CDCl₃) δ 12.09 (s, 1H), 8.82 (d, J = 9.0 Hz, 1H), 8.28 (s, 2H), 7.55 (s, 1H), 7.48 (s, 1H), 7.42 (d, J = 9.8 Hz, 1H), 7.28 (s, 1H), 7.15 (dd, J = 8.9, 2.2 Hz, 1H), 6.47 (d, J = 16.7 Hz, 1H), 6.30 (dd, J = 16.7, 10.2 Hz, 1H), 5.79 (d, J = 10.2 Hz, 1H), 3.75 (s, 3H).

### Example 37: Preparation of N-(4-((4-chloro-5-(trifluoromethyl)pyridin-2-yl)amino)-3-(1-methyl-1H-pyrazol-3-yl)phenyl)acrylamide

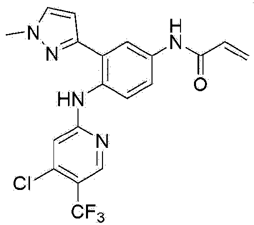

The title compound (10 mg, yield: 24%) was obtained in the same manner as in Example 4, except that 1-methyl-4-(tributylstannyl)-1H-pyrazole was used instead of 2-(tributylstannyl)pyridine in step 1 of Example 4, and 4-chloro-5-(trifluoromethyl)pyridin-2-amine was used instead of 5-(trifluoromethyl)pyridin-2-amine in step 2 of Example 4.

¹H NMR (500 MHz, CDCl₃) δ 10.71 (s, 1H), 8.47 (s, 1H), 8.38 (d, J = 8.8 Hz, 1H), 8.18 (s, 1H), 7.45 (d, J = 2.1 Hz, 1H), 7.37 (d, J = 7.7 Hz, 1H), 6.88 (s, 1H), 6.68 (d, J = 1.8 Hz, 1H), 6.48 (d, J = 16.8 Hz, 1H), 6.29 (dd, J = 16.8, 10.2 Hz, 1H), 5.82 (d, J = 10.2 Hz, 1H), 4.04 (s, 3H) one proton is missing due to overlapping.

### Example 38: Preparation of N-(3-fluoro-5-(1-methyl-1H-imidazol-4-yl)-4-((5-(trifluoromethyl)pyridin-2-yl)amino)phenyl)acrylamide

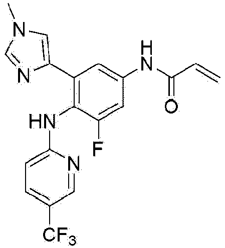

The title compound (8 mg, yield: 20%) was obtained in the same manner as in Example 4, except that 1-bromo-2,3-difluoro-5-nitrobenzene and 1-methyl-4-(tributylstannyl)-1H-imidazole were respectively used instead of 2-bromo-1-fluoro-4-nitrobenzene and 2-(tributylstannyl)pyridine in step 1 of Example 4.

¹H NMR (500 MHz, CDCl₃) δ 9.00 (s, 1H), 8.88 (s, 1H), 8.40 (s, 1H), 8.08 (s, 1H), 7.95 (s, 1H), 7.81 (d, J = 9.5 Hz, 1H), 7.59 - 7.51 (m, 2H), 6.51 (d, J = 16.8 Hz, 1H), 6.36 (d, J = 10.2 Hz, 1H), 5.84 (d, J = 10.1 Hz, 1H), 3.85 (s, 3H) one proton is missing due to overlapping.

### Example 39: Preparation of N-(2'-((5-(trifluoromethyl)pyridin-2-yl)amino)-[2,3'-bipyridin]-5'-yl)acrylamide

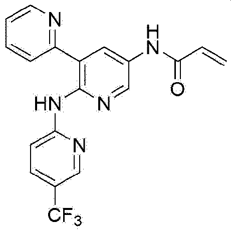

The title compound (7 mg, yield: 18%) was obtained in the same manner as in Example 1, except that 5-(trifluoromethyl)pyridin-2-amine and 3-bromo-2-chloro-5-nitropyridine were respectively used instead of cyclohexanamine and 2-bromo-1-fluoro-4-nitrobenzene in step 1 of Example 1, and 1-methyl-4-(tributylstannyl)-1H-imidazole was used instead of phenylboronic acid in step 2 of Example 1.

¹H NMR (500 MHz, CDCl₃) δ 12.42 (s, 1H), 8.80 (d, J = 4.7 Hz, 1H), 8.74 (d, J = 2.2 Hz, 1H), 8.53 (d, J = 7.2 Hz, 2H), 8.30 (d, J = 2.4 Hz, 1H), 7.91 (s, 1H), 7.82 (t, J = 7.9 Hz, 3H), 7.32 (d, J = 4.0 Hz, 1H), 6.51 (d, J = 16.8 Hz, 1H), 6.35 (dd, J = 16.8, 10.2 Hz, 1H), 5.84 (d, J = 10.3 Hz, 1H) one proton is missing due to overlapping

### Example 40: Preparation of N-(4-((3-fluoro-4-(trifluoromethyl)phenyl)amino)-3-(1-methyl-1H-imidazol-4-yl)phenyl)acrylamide

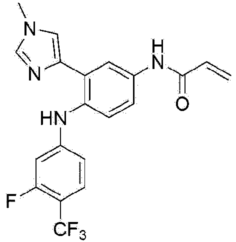

The title compound (6 mg, yield: 15%) was obtained in the same manner as in Example 35, except that (3-fluoro-4-(trifluoromethyl)phenyl)boronic acid was used instead of 4-(trifluoromethyl)phenyl)boronic acid in step 2 of Example 35.

1H NMR (500 MHz, CDCl₃) δ 10.17 (s, 1H), 8.11 (d, J = 2.0 Hz, 1H), 7.51 (s, 1H), 7.46 - 7.34 (m, 2H), 7.25 (s, 1H), 7.20 (dd, J = 8.6, 2.0 Hz, 1H), 6.90 (t, J = 11.6 Hz, 2H), 6.48 (d, J = 16.8 Hz, 1H), 6.30 (d, J = 10.2 Hz, 1H), 5.81 (d, J = 10.2 Hz, 1H), 3.76 (s, 3H) ) one proton is missing due to overlapping.

### Example 41: Preparation of N-(5-(1-methyl-1H-imidazol-4-yl)-6-((4-(trifluoromethyl)phenyl)amino)pyridin-3-yl)acrylamide

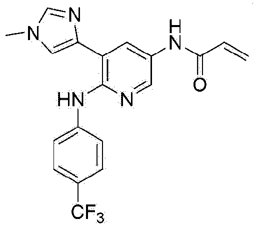

The title compound (6 mg, yield: 15%) was obtained in the same manner as in Example 35, except that 3-bromo-5-nitropyridin-2-amine was used instead of 2-bromo-4-nitroaniline in step 2 of Example 35.

¹H NMR (500 MHz, MeOD) δ 8.29 (dd, J = 15.9, 2.5 Hz, 2H), 7.88 (d, *J* = 8.6 Hz, 2H), 7.76 (s, 1H), 7.59 - 7.49 (m, 3H), 6.43 (dd, J = 15.0, 5.9 Hz, 2H), 5.82 (dd, J = 9.6, 2.1 Hz, 1H), 3.83 (s, 3H).

### Example 42: Preparation of N-(3-(1-methyl-1H-pyrazol-3-yl)-4-((4-(trifluoromethyl)phenyl)amino)phenyl)acrylamide

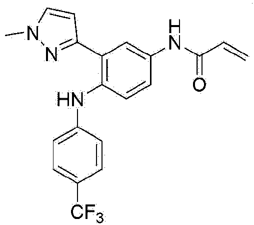

The title compound (7 mg, yield: 18%) was obtained in the same manner as in Example 35, except that 1-methyl-4-(tributylstannyl)-1H-pyrazole was used instead of 1-methyl-4-(tributylstannyl)-1H-imidazole in step 1 of Example 35.

¹H NMR (500 MHz, DMSO) δ 10.21 (s, 1H), 9.03 (s, 1H), 8.11 (d, *J* = 2.3 Hz, 1H), 7.76 (d, *J* = 2.2 Hz, 1H), 7.68 (dd, *J* = 8.7, 2.3 Hz, 1H), 7.48 (d, *J* = 8.5 Hz, 2H), 7.37 (d, *J* = 8.7 Hz, 1H), 7.01 (d, *J* = 8.5 Hz, 2H), 6.53 (d, *J* = 2.2 Hz, 1H), 6.45 (dd, *J* = 17.0, 10.1 Hz, 1H), 6.27 (dd, *J =* 17.0, 1.8 Hz, 1H), 5.76 (dd, *J =* 10.1, 1.8 Hz, 1H), 3.92 (s, 3H).

### Example 43: Preparation of N-(4-((3-chloro-4-(trifluoromethyl)phenyl)amino)-3-(1-methyl-1H-imidazol-4-yl)phenyl)acrylamide

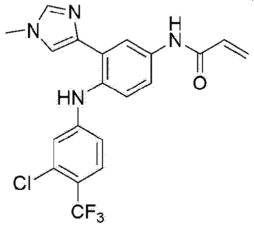

The title compound (10 mg, yield: 24%) was obtained in the same manner as in Example 35, except that (3-chloro-4-(trifluoromethyl)phenyl)boronic acid was used instead of 4-(trifluoromethyl)phenyl)boronic acid in step 2 of Example 35.

¹H NMR (500 MHz, CDCl₃) δ 10.14 (s, 1H), 8.11 (s, 1H), 7.58 - 7.34 (m, 4H), 7.23 (d, *J =* 19.3 Hz, 3H), 7.04 - 6.99 (m, 1H), 6.47 (d, *J =* 16.8 Hz, 1H), 6.29 (dd, *J =* 16.8, 10.2 Hz, 1H), 5.80 (d, *J =* 10.2 Hz, 1H), 3.75 (s, 3H).

### Example 44: Preparation of N-(4-((2,3-difluoro-4-(trifluoromethyl)phenyl)amino)-3-(1-methyl-1H-imidazol-4-yl)phenyl)acrylamide

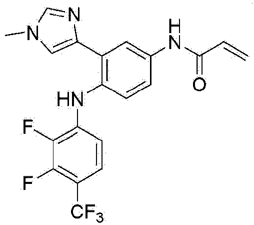

The title compound (8 mg, yield: 19%) was obtained in the same manner as in Example 35, except that (2,3-difluoro-4-(trifluoromethyl)phenyl)boronic acid was used instead of 4-(trifluoromethyl)phenyl)boronic acid in step 2 of Example 35.

¹H NMR (500 MHz, CDCl₃) δ 10.18 (s, 1H), 8.06 (d, *J =* 1.2 Hz, 1H), 7.63 (s, 1H), 7.51 (s, 1H), 7.39 (d, *J* = 8.7 Hz, 1H), 7.29 - 7.24 (m, 1H), 7.22 (s, 1H), 7.12 (t, *J* = 4.9 Hz, 2H), 6.47 (d, *J* = 16.7 Hz, 1H), 6.30 (dd, *J* = 16.8, 10.2 Hz, 1H), 5.79 (d, *J =* 10.3 Hz, 1H), 3.73 (s, 3H).

### Example 45: Preparation of N-(3-(1-methyl-1H-imidazol-4-yl)-4-((4-(trifluoromethoxy)phenyl)amino)phenyl)acrylamide

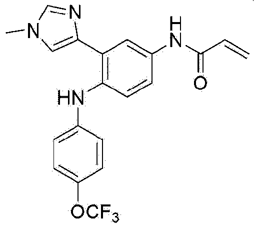

The title compound (8 mg, yield: 20%) was obtained in the same manner as in Example 35, except that (4-(trifluoromethoxy)phenyl)boronic acid was used instead of 4-(trifluoromethyl)phenyl)boronic acid in step 2 of Example 35.

¹H NMR (500 MHz, CDCl₃) δ 10.18 (s, 1H), 8.06 (d, *J =* 1.2 Hz, 1H), 7.63 (s, 1H), 7.51 (s, 1H), 7.39 (d, *J* = 8.7 Hz, 1H), 7.29 - 7.24 (m, 1H), 7.22 (s, 1H), 7.12 (t, *J* = 4.9 Hz, 2H), 6.47 (d, *J* = 16.7 Hz, 1H), 6.30 (dd, *J* = 16.8, 10.2 Hz, 1H), 5.79 (d, *J* = 10.3 Hz, 1H), 3.73 (s, 3H).

¹H NMR (500 MHz, CDCl₃) δ 9.78 (s, 1H), 8.04 (d, *J* = 2.1 Hz, 1H), 7.87 (s, 1H), 7.72 - 7.66 (m, 2H), 7.57 (td, J = 7.4, 1.2 Hz, 1H), 7.51 - 7.45 (m, 2H), 7.29 (d, *J* = 4.9 Hz, 1H), 7.17 - 7.13 (m, 2H), , 6.43 (d, *J* = 16.6 Hz, 1H), 6.35 -6.25 (m, 1H), 5.73 (dd, *J =* 10.1, 1.1 Hz, 1H), 3.69 (s, 3H).

### Example 46: Preparation of N-(4-((3,5-difluoro-4-(trifluoromethyl)phenyl)amino)-3-(1-methyl-1H-imidazol-4-yl)phenyl)acrylamide

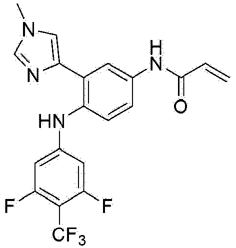

The title compound (5 mg, yield: 12%) was obtained in the same manner as in Example 35, except that (3,5-difluoro-4-(trifluoromethyl)phenyl)boronic acid was used instead of 4-(trifluoromethyl)phenyl)boronic acid in step 2 of Example 35.

¹H NMR (500 MHz, CDCl₃) δ 10.32 (s, 1H), 8.14 (s, 1H), 7.49 (d, *J* = 11.9 Hz, 2H), 7.39 (d, *J* = 8.7 Hz, 1H), 7.24 (s, 1H), 7.21 (s, 1H), 6.65 (d, *J* = 12.0 Hz, 2H), 6.47 (d, *J* = 16.7 Hz, 1H), 6.30 (s, 1H), 5.80 (d, *J* = 10.4 Hz, 1H), 3.75 (s, 3H).

### Example 47: Preparation of N-(4-((4-chlorophenyl)amino)-3-(1-methyl-1H-imidazol-4-yl)phenyl)acrylamide

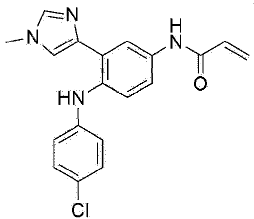

The title compound (15 mg, yield: 42%) was obtained in the same manner as in Example 35, except that (4-chlorophenyl)boronic acid was used instead of 4-(trifluoromethyl)phenyl)boronic acid in step 2 of Example 35.

¹H NMR (500 MHz, CDCl₃) δ 9.69 (s, 1H), 8.03 (dd, J = 9.6, 2.0 Hz, 1H), 7.54 (s, 1H), 7.47 (s, 1H), 7.29 (d, J = 8.9 Hz, 1H), 7.23 - 7.16 (m, 3H), 7.16 - 7.12 (m, 1H), 7.10 (d, *J* = 8.6 Hz, 2H), 6.44 (d, *J* = 16.8 Hz, 1H), 6.28 (dd, J = 16.8, 10.2 Hz, 1H), 5.76 (t, *J* = 8.1 Hz, 1H), 3.71 (s, 3H).

### Example 48: Preparation of N-(4-((5-bromo-6-methylpyridin-2-yl)amino)-3-(1-methyl-1H-imidazol-4-yl)phenyl)acrylamide

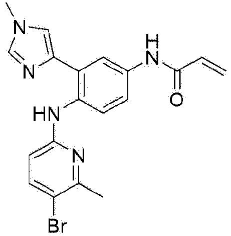

The title compound (7 mg, yield: 17%) was obtained in the same manner as in Example 4, except that 1-methyl-4-(tributylstannyl)-1H-imidazole was used instead of 2-(tributylstannyl)pyridine in step 1 of Example 4, and 5-bromo-6-methylpyridin-2-amine was used instead of 5-(trifluoromethyl)pyridin-2-amine in step 2 of Example 4.

¹H NMR (500 MHz, CDCl₃) δ 10.76 (s, 1H), 8.38 (d, J = 8.9 Hz, 1H), 8.12 (d, *J* = 2.0 Hz, 1H), 7.63 - 7.39 (m, 3H), 7.25 - 7.17 (m, 2H), 6.57 (d, *J* = 8.7 Hz, 1H), 6.45 (d, *J =* 16.7 Hz, 1H), 6.31 (d, *J =* 10.2 Hz, 1H), 5.77 (d, *J =* 10.2 Hz, 1H), 3.71 (s, 3H), 2.58 (s, 3H).

### Example 49: Preparation of N-(4-((5-chloro-6-methylpyridin-2-yl)amino)-3-(1-methyl-1H-imidazol-4-yl)phenyl)acrylamide

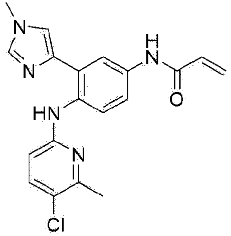

The title compound (10 mg, yield: 27%) was obtained in the same manner as in Example 4, except that 1-methyl-4-(tributylstannyl)-1H-imidazole was used instead of 2-(tributylstannyl)pyridine in step 1 of Example 4, and 5-chloro-6-methylpyridin-2-amine was used instead of 5-(trifluoromethyl)pyridin-2-amine in step 2 of Example 4.

¹H NMR (500 MHz, CDCl₃) δ 10.71 (s, 1H), 8.38 (d, J = 8.9 Hz, 1H), 8.13 (d, *J* = 2.1 Hz, 1H), 7.49 (d, *J* = 15.4 Hz, 2H), 7.37 (d, *J* = 8.7 Hz, 1H), 7.28 - 7.15 (m, 2H), 6.64 (d, *J* = 8.7 Hz, 1H), 6.45 (d, *J* = 16.6 Hz, 1H), 6.29 (dd, *J* = 16.8, 10.2 Hz, 1H), 5.77 (d, *J* = 10.2 Hz, 1H), 3.73 (s, 3H), 2.54 (s, 3H).

### Example 50: Preparation of N-(4-((5-chloropyridin-2-yl)amino)-3-(1-methyl-1H-imidazol-4-yl)phenyl)acrylamide

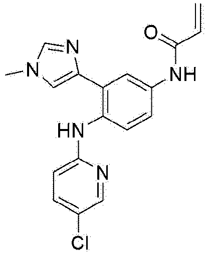

The title compound (12 mg, yield: 28%) was obtained in the same manner as in Example 4, except that 1-methyl-4-(tributylstannyl)-1H-imidazole was used instead of 2-(tributylstannyl)pyridine in step 1 of Example 4, and 4-chloropyridin-2-amine was used instead of 5-(trifluoromethyl)pyridin-2-amine in step 2 of Example 4.

¹H NMR (500 MHz, CDCl₃) δ 10.94 (s, 1H), 8.31 (d, J = 8.8 Hz, 1H), 8.17 (dd, *J =* 6.9, 2.2 Hz, 2H), 7.51 (s, 1H), 7.42 (dd, *J* = 9.0, 2.4 Hz, 2H), 7.26 (s, 1H), 7.18 (dd, *J* = 8.8, 2.1 Hz, 1H), 6.80 (d, *J* = 8.8 Hz, 1H), 6.46 (d, *J =* 16.8 Hz, 1H), 6.28 (dd, J *=* 16.8, 10.2 Hz, 1H), 5.78 (d, *J =* 10.2 Hz, 1H), 3.74 (s, 3H).

### Example 51: Preparation of N-(4-((5-bromo-4-chloropyridin-2-yl)amino)-3-(1-methyl-1H-imidazol-4-yl)phenyl)acrylamide

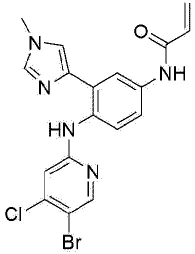

The title compound (10 mg, yield: 23%) was obtained in the same manner as in Example 4, except that 1-methyl-4-(tributylstannyl)-1H-imidazole was used instead of 2-(tributylstannyl)pyridine in step 1 of Example 4, and 5-bromo-4-chloropyridin-2-amine was used instead of 5-(trifluoromethyl)pyridin-2-amine in step 2 of Example 4.

¹H NMR (500 MHz, CDCl₃) δ 11.08 (s, 1H), 8.29 (s, 1H), 8.22 (d, J = 8.8 Hz, 1H), 8.16 (d, J = 2.1 Hz, 1H), 7.73 (s, 1H), 7.49 (s, 1H), 7.25 - 7.14 (m, 2H), 6.95 (s, 1H), 6.45 (d, J = 16.7 Hz, 1H), 6.30 (dd, J = 16.8, 10.2 Hz, 1H), 5.77 (d, J = 10.3 Hz, 1H), 3.71 (s, 3H).

### Example 52: Preparation of N-(4-((5-bromopyridin-2-yl)amino)-3-(1-methyl-1H-imidazol-4-yl)phenyl)acrylamide

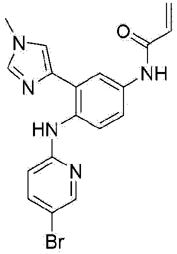

The title compound (5 mg, yield: 13%) was obtained in the same manner as in Example 4, except that 1-methyl-4-(tributylstannyl)-1H-imidazole was used instead of 2-(tributylstannyl)pyridine in step 1 of Example 4, and 5-bromopyridin-2-amine was used instead of 5-(trifluoromethyl)pyridin-2-amine in step 2 of Example 4.

¹H NMR (500 MHz, CDCl₃) δ 10.91 (s, 1H), 8.31 (d, *J* = 8.8 Hz, 1H), 8.25 (d, *J* = 2.2 Hz, 1H), 8.18 (d, J = 2.0 Hz, 1H), 7.53 (dd, *J =* 10.2, 3.6 Hz, 2H), 7.38 (s, 1H), 7.27 (s, 1H), 7.17 (dd, J = 8.8, 2.1 Hz, 1H), 6.76 (d, *J* = 8.8 Hz, 1H), 6.46 (d, *J* = 16.7 Hz, 1H), 6.28 (dd, J = 16.8, 10.2 Hz, 1H), 5.78 (d, *J =* 10.2 Hz, 1H), 3.75 (s, 3H).

### Example 53: Preparation of N-(4-((3-chloro-5-(trifluoromethyl)pyridin-2-yl)amino)-3-(1-methyl-1H-pyrazol-3-yl)phenyl)acrylamide

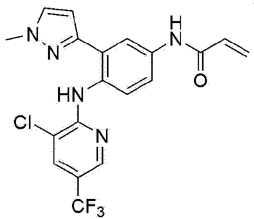

The title compound (3 mg, yield: 7%) was obtained in the same manner as in Example 4, except that 1-methyl-4-(tributylstannyl)-1H-imidazole was used instead of 2-(tributylstannyl)pyridine in step 1 of Example 4, and 3-chloro-5-(trifluoromethyl)pyridin-2-amine was used instead of 5-(trifluoromethyl)pyridin-2-amine in step 2 of Example 4.

¹H NMR (500 MHz, DMSO) δ 11.36 (s, 1H), 10.19 (s, 1H), 8.63 (d, J = 9.0 Hz, 1H), 8.50 (s, 1H), 8.25 (s, 1H), 8.10 (s, 1H), 7.88 (s, 1H), 7.59 (d, J = 9.1 Hz, 1H), 6.62 (d, J = 2.2 Hz, 1H), 6.48-6.38 (m, 1H), 6.26 (d, J = 17.0 Hz, 1H), 5.75 (d, J = 10.2 Hz, 1H), 3.96 (s, 3H).

### Example 54: Preparation of N-(3-(1-methyl-1H-pyrazol-3-yl)-4-((4-(trifluoromethyl)pyridin-2-yl)amino)phenyl)acrylamide

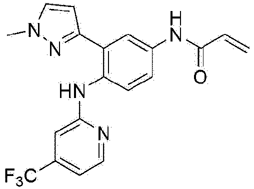

The title compound (15 mg, yield: 39%) was obtained in the same manner as in Example 4, except that 1-methyl-4-(tributylstannyl)-1H-pyrazole was used instead of 2-(tributylstannyl)pyridine in step 1 of Example 4, and 4-(trifluoromethyl)pyridin-2-amine was used instead of 5-(trifluoromethyl)pyridin-2-amine in step 2 of Example 4.

¹H NMR (500 MHz, DMSO) δ 10.19 (s, 1H), 10.17 (s, 1H), 8.35 (d, J = 5.2 Hz, 1H), 8.13 (d, J = 8.9 Hz, 1H), 8.07 (d, J = 2.2 Hz, 1H), 7.81 (d, J = 2.1 Hz, 1H), 7.60 (dd, J = 8.9, 2.2 Hz, 1H), 7.04 (s, 1H), 7.00 (d, J = 5.2 Hz, 1H) 6.55 (d, J = 2.1 Hz, 1H), 6.44 (dd, J = 16.9, 10.1 Hz, 1H), 6.26 (dd, J = 17.0, 1.7 Hz, 1H), 5.77-5.74 (m, 1H), 3.96 (s, 3H).

### Example 55: Preparation of N-(4-((4,5-dichloropyridin-2-yl)amino)-3-(1-methyl-1H-pyrazol-3-yl)phenyl)acrylamide

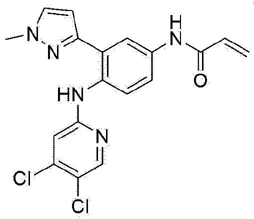

The title compound (15 mg, yield: 39%) was obtained in the same manner as in Example 4, except that 1-methyl-4-(tributylstannyl)-1H-pyrazole was used instead of 2-(tributylstannyl)pyridine in step 1 of Example 4, and 3,4-dichloropyridin-2-amine was used instead of 5-(trifluoromethyl)pyridin-2-amine in step 2 of Example 4.

¹H NMR (500 MHz, DMSO) δ 10.15 (s, 1H), 10.11 (s, 1H), 8.26 (s, 1H), 8.06-8.04 (m, 2H), 7.80 (d, J = 2.2 Hz, 1H), 7.58 (dd, J = 8.9, 2.3 Hz, 1H), 7.07 (s, 1H), 6.54 (d, J = 2.2 Hz, 1H), 6.42 (dd, J = 16.9, 10.1 Hz, 1H), 6.24 (dd, J = 17.0, 1.8 Hz, 1H), 5.73 (dd, J = 10.1, 1.8 Hz, 1H), 3.96 (s, 3H)

### Example 56: Preparation of N-(4-((3-fluoro-5-(trifluoromethyl)pyridin-2-yl)amino)-3-(1-methyl-1H-pyrazol-3-yl)phenyl)acrylamide

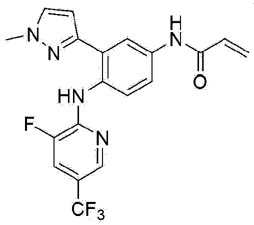

The title compound (14 mg, yield: 35%) was obtained in the same manner as in Example 4, except that 1-methyl-4-(tributylstannyl)-1H-pyrazole was used instead of 2-(tributylstannyl)pyridine in step 1 of Example 4, and 2-fluoro-4-(trifluoromethyl)pyridin-2-amine was used instead of 5-(trifluoromethyl)pyridin-2-amine in step 2 of Example 4.

¹H NMR (500 MHz, CDCl₃) δ 11.59 (s, 1H), 8.93 (d, J = 8.9 Hz, 1H), 8.33 (s, 1H), 8.26 (s, 1H), 7.46 (t, J = 6.4 Hz, 2H), 7.35 (d, J = 7.2 Hz, 1H), 6.72 (s, 1H), 6.49 (d, J = 16.8 Hz, 1H), 6.30 (dd, J = 16.8, 10.2 Hz, 1H), 5.82 (d, J = 10.2 Hz, 1H), 4.03 (s, 3H), one proton is missing due to overlapping.

### Experimental Example 1: Activity inhibition test of transcription factor TEAD

The transcription factor TEAD activity of the compounds prepared in Examples was measured by the ONE-Glo^{™} Luciferase assay (Promega, Catalog #E6110) method using the Hippo Pathway TEAD reporter-MCF7 recombinant cell line (BPS Bioscience, Catalog #60618).

Specifically, the TEAD Reporter-MCF7 cell line contains the firefly luciferase gene whose expression is regulated under the control of TEAD response elements in the MCF7, which is a human breast cancer cell line. In the cell line, a non-phosphorylated YAP/TAZ exists in the nucleus in a non-stressed condition, which continuously induces expression of the luciferase reporter.

TEAD Reporter-MCF7 cells were prepared in 100 µL of cell culture medium (MEM medium, 10% FBS, 1% P/S, 400 µg/mL Geneticin, 1% N-ethyl acetate A, 1 mM NA pyruvate, 10 µg/mL Insulin) in a white clear bottom 96-well microplate. The prepared plate was incubated in a 5% CO₂ incubator at 37°C for 24 hours, and then the previously prepared compounds of Examples were finally treated at concentrations of 0.001, 0.01, 0.1, 1, and 10 µM, which were repeated three times for all treatment solutions. The plates treated with the compounds of Examples were incubated in a 5% CO₂ incubator at 37°C for 24 hours. 100 µL/well of the substrate aqueous solution containing luciferin was added to a white clear-bottom 96 well microplate, thereby initiating an enzyme reaction. The reaction was performed at room temperature for 5 minutes, and the luminescence (integration time 1000 ms) was measured using a Flexstation3 multi-mode microplate reader. In accordance with the instructions for the ONE-Glo^{™} Luciferase Assay, the luciferase enzyme activity, which represents the activity of the transcription factor TEAD, was measured by a chemiluminescence method, and the inhibitory activity of the compound according to the present invention was calculated. The results of each compound were analyzed using Microsoft Excel, and the IC₅₀ values were calculated by GraphPad Prism software. The results are shown in Table 1 below.

### Experimental Example 2: Cell growth inhibition test

The cell growth for the compounds prepared in Examples was measured by CellTiter-Glo^{®} Luminescednt Cell Viability (Promega, Catalog #G7571) method using NCI-H226 (addexbio), NCI-H28 (Korea Cell Line Bank) and MSTO-211H (elabscience) human mesothelioma cell lines. This evaluation method is a method in which when luciferase enzyme is activated by ATP flowing out from living cells, it reacts with a luciferin substrate, and the enzyme activity at this time is measured to confirm cell viability.

Specifically, NCI-H226, NCI-H28, and MSTO-211H cells were prepared in 100 µL of cell culture medium (RPMI medium, 10% FBS, 1% P/S, 4.5 g/L D-Glucose, 2.383 g/L HEPES Buffer, L-Glutamine, 1.5 g/L Sodium Bicarbonate, 110 mg/L Sodium pyruvate) in a white clear bottom 96-well microplate. The prepared plate was incubated in a 5% CO₂ incubator at 37°C for 24 hours, and then the previously prepared compounds of Examples were finally treated at concentrations of 0.001, 0.01, 0.1, 1, and 10 µM, which was repeated three times for all treatment solutions. The plates treated with the compounds of Examples were incubated in a 5% CO₂ incubator at 37°C. 100 µL/well of the substrate aqueous solution containing luciferin was added to a white clear-bottom 96 well microplate, thereby initiating an enzyme reaction. The reaction was performed at room temperature in the dark for 10 minutes, and the luminescence was measured using a Flexstation3 multi-mode microplate reader. In accordance with the instructions for the CellTiter-Glo^{™} Luminescednt Cell Viability, the luciferase enzyme activity, which represents the amount of ATP, was measured by a chemiluminescence method, and the inhibitory activity of the compounds according to the present invention was calculated. The results of each compound were analyzed using Microsoft Excel, and the IC₅₀ values were calculated using GraphPad Prism software. The results are shown in Tables 1 and 2 below.

**[Table 1]**

| Example No. | TEAD IC₅₀ (nM) | Growth IC₅₀ (nM) H226 |
|---|---|---|
| 1 | 42 | 132 |
| 2 | 12.3 | 46 |
| 3 | 3.7 | >10,000 |
| 4 | 17.8 | 6 |
| 6 | 10 | 3 |
| 7 | 53.5 | 122 |
| 8 | 15.4 | 14 |
| 9 | 80 | 26 |
| 10 | 23 | 10 |
| 11 | 28.2 | 129 |
| 12 | 2.4 | 1,250 |
| 15 | 42.8 | 71.8 |
| 16 | 69.1 | 148 |
| 21 | 11.5 | 9 |
| 23 | 48 | 55 |
| 27 | 30 | 985 |
| 28 | 28.6 | 875 |
| 29 | 21.5 | 20 |
| 30 | 31.6 | 10 |
| 31 | 314 | N/D |
| 34 | 5.7 | 31.4 |
| 35 | 5.3 | 3 |
| 36 | 27 | 12 |
| 37 | 4.5 | 13 |
| 39 | 40.3 | 15.8 |
| 40 | 15 | 6.6 |
| 41 | 10 | 6 |
| 42 | 7 | 4.7 |
| 43 | 33 | 7.4 |
| 44 | 8 | 3.9 |
| 45 | 113 | 33.8 |
| 46 | 28 | 13.1 |
| 47 | 3.9 | 3.3 |
| 48 | 45 | 10.3 |
| 49 | 258 | 18.8 |
| 50 | 88 | 10.4 |
| 53 | 30.7 | 62.8 |
| 54 | 23.4 | 11.8 |
| 55 | 12.8 | 65 |
| 56 | 5 | 11 |

**[Table 2]**

| Example No. | growth IC₅₀ (nM) MSTO-211H | Growth IC₅₀ (nM) H28 |
|---|---|---|
| 4 | 129.7 | 3,310 |
| 6 | 220.9 | 1,380 |
| 15 | 551.5 | 1,571 |
| 21 | 3,270 | 2,679 |
| 29 | 196.5 | 2,550 |
| 30 | 2,210 | 8,790 |
| 31 | >10,000 | 3,111 |
| 35 | 104.7 | 3,015 |
| 36 | >10,000 | >10,000 |
| 37 | 3,750 | 1,097 |
| 39 | 194.2 | 3,277 |
| 40 | 1,130 | 5,537 |
| 53 | 8,240 | 1,667 |
| 55 | 166.7 | 1,246 |

## Claims

1. A compound represented by the following Chemical Formula 1 or 2, or a pharmaceutically acceptable salt thereof: 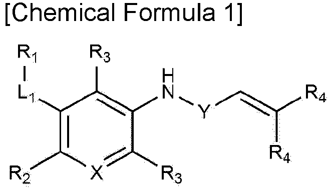 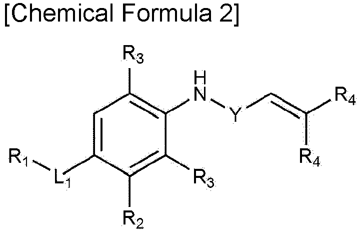 in Chemical Formulas 1 and 2,
L₁ is a single bond, a C₁₋₆ alkylene, a C₂₋₄ alkenylene, or a C₂₋₄ alkynylene,
R₁ is phenyl, or a 5- or 6-membered heterocycle containing 1 to 4 heteroatoms each independently selected from the group consisting of N, O and S,
the R₁ is unsubstituted or substituted with halogen, a C₁₋₄ alkyl, a C₁₋₄ thioalkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, a C₃₋₆ cycloalkyl, amino, nitro, cyano, a (C₁₋₄ alkyl)amino, or a di(C₁₋₄ alkyl)amino,
R₂ is -N(R₉)-L₂-R₅, 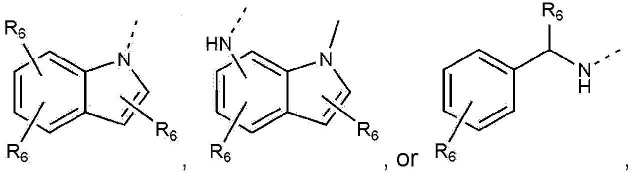
L₂ is a single bond, a C₁₋₆ alkylene, a C₂₋₄ alkenylene, or a C₂₋₄ alkynylene,
R₅ is a C₃₋₇ cycloalkyl, phenyl, or a 5- or 6-membered heteroaryl containing 1 to 4 heteroatoms each independently selected from the group consisting of N, O and S,
the R₅ is unsubstituted or substituted with 1 to 3 substituents each independently selected from the group consisting of hydroxy, halogen, a C₁₋₄ alkyl, a C₁₋₄ alkoxy, a C₁₋₄ haloalkyl, a C₁₋₄ haloalkoxy, a C₁₋₄ thioalkyl, and a C₃₋₆ cycloalkyl,
each R₆ is independently hydrogen, halogen, a C₁₋₆ alkyl, a C₁₋₆ haloalkyl, a C₁₋₆ alkoxy, or a C₃₋₇ cycloalkyl,
R₉ is hydrogen, halogen, a C₁₋₄ alkyl, a C₁₋₄ alkoxy, a C₁₋₄ haloalkyl, a C₁₋₄ haloalkoxy, or a C₃₋₆ cycloalkyl,
each R₃ is independently hydrogen, halogen, a C₁₋₆ alkyl, a C₁₋₆ haloalkyl, or a C₁₋₄ alkoxy,
each R₄ is independently hydrogen, a C₁₋₄ alkyl, -CH₂N(R₇)₂, or a 5- or 6-membered heteroaliphatic ring containing one or two N which is substituted with R₇,
each R₇ is independently hydrogen, or a C₁₋₄alkyl,
X is CRs, or N,
R₈ is hydrogen, or halogen, and
Y is CO, CS, or SO₂.

2. The compound or the pharmaceutically acceptable salt thereof according to claim 1, wherein:
L₁ is a single bond, or -CH=CH-.

3. The compound or the pharmaceutically acceptable salt thereof according to claim 1, wherein:
R₁ is phenyl, pyridinyl, pyrimidinyl, imidazolyl, pyrazolyl, triazolyl, tetrazolyl, oxadiazolyl, pyrrolidinyl, imidazolyl, pyrazolyl, oxazolyl, isoxazolyl, thiazolyl, or isothiazolyl,
the R₁ is unsubstituted or substituted with halogen, a C₁₋₄ alkyl, a C₁₋₄ thioalkyl, a C₁₋₄ haloalkyl, a C₁₋₄ alkoxy, a C₁₋₄ haloalkoxy, amino, nitro, cyano, a (C₁₋₄ alkyl)amino, or a di(C₁₋₄ alkyl)amino.

4. The compound or the pharmaceutically acceptable salt thereof according to claim 1, wherein:
L₂ is a single bond, methylene, or ethylene.

5. The compound or the pharmaceutically acceptable salt thereof according to claim 1, wherein:
R₅ is cyclopentyl, cyclohexyl, phenyl, pyridinyl, pyrimidinyl, or thiazolyl,
the R₅ is unsubstituted or substituted with 1 to 3 substituents each independently selected from the group consisting of hydroxy, halogen, a C₁₋₄ alkyl, a C₁₋₄ alkoxy, a C₁₋₄ haloalkyl, a C₁₋₄ haloalkoxy, a C₁₋₄ thioalkyl, and a C₃₋₆ cycloalkyl.

6. The compound or the pharmaceutically acceptable salt thereof according to claim 1, wherein:
each R₆ is independently hydrogen, fluoro, chloro, difluoromethyl, or trifluoromethyl.

7. The compound or the pharmaceutically acceptable salt thereof according to claim 1, wherein:
R₉ is hydrogen.

8. The compound or the pharmaceutically acceptable salt thereof according to claim 1, wherein:
each R₃ is independently hydrogen, or methoxy.

9. The compound or the pharmaceutically acceptable salt thereof according to claim 1, wherein:
R₄ is all hydrogen, or
one of R₄ is hydrogen, and the other is -CH₂N(R₇)₂, or a 5- or 6-membered heteroaliphatic ring containing one or two N which is substituted with R₇.

10. The compound or the pharmaceutically acceptable salt thereof according to claim 1, wherein:
X is CH, CF, or N.

11. The compound or the pharmaceutically acceptable salt thereof according to claim 1, wherein
the compound is a compound represented by the following Chemical Formula 3: 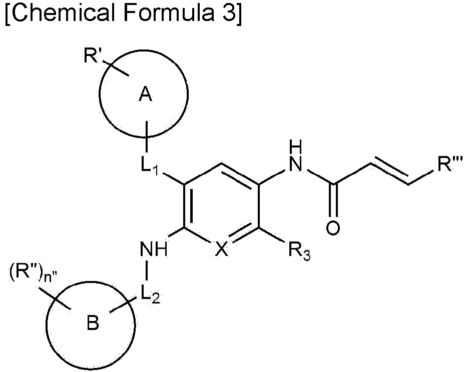
in Chemical Formula 3,
X is CH, CF, or N,
A is benzene, pyridine, pyrimidine, imidazole, pyrazole, triazole, tetrazole, or oxadiazole,
R' is hydrogen, halogen, a C₁₋₄ alkyl, or a C₁₋₄ alkoxy,
L₁ is a single bond, a C₁₋₆ alkylene, or a C₂₋₄ alkenylene,
B is benzene, pyridine, pyrimidine, thiazolyl, cyclopentyl, or cyclohexyl,
each R" is independently hydrogen, hydroxy, halogen, cyano, a C₁₋₄ alkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, or a C₃₋₆ cycloalkyl,
n" is an integer of 1 to 3,
L₂ is a single bond, or a C₁₋₆ alkylene,
R‴ is hydrogen, or -CH₂-N(CH₃)₂, and
R₃ is hydrogen or a C₁₋₄ alkoxy.

12. The compound or the pharmaceutically acceptable salt thereof according to claim 1, wherein
the compound is a compound represented by the following Chemical Formula 4: 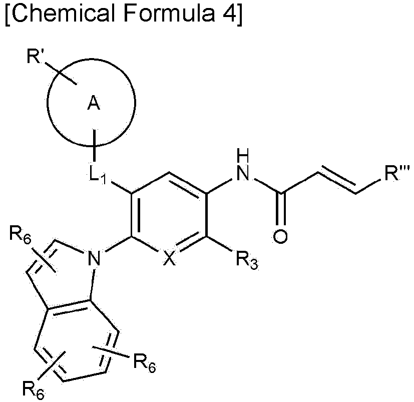
in Chemical Formula 4,
X is CH, CF, or N,
A is benzene, pyridine, pyrimidine, imidazole, pyrazole, triazole, tetrazole, or oxadiazole,
R' is hydrogen, halogen, a C₁₋₄ alkyl, or a C₁₋₄ alkoxy,
L₁ is a single bond, a C₁₋₆ alkylene, or a C₂₋₄ alkenylene,
each R₆ is independently hydrogen, halogen, a C₁₋₆ alkyl, a C₁₋₆ haloalkyl, a C₁₋₆ alkoxy, or a C₃₋₇ cycloalkyl,
R₃ is hydrogen, or a C₁₋₄ alkoxy, and
R‴ is hydrogen, or -CH₂-N(CH₃)₂.

13. The compound or the pharmaceutically acceptable salt thereof according to claim 1, wherein
the compound is any one selected from the group consisting of:
1) N-(6-(cyclohexylamino)-[1,1'-biphenyl]-3-yl)acrylamide,
2) N-(4-(cyclohexylamino)-3-(pyridin-2-yl)phenyl)acrylamide,
3) N-(3-(pyridin-2-yl)-4-((cis-4-(trifluoromethyl)cyclohexyl)amino)phenyl)acrylamide,
4) N-(3-(pyridin-2-yl)-4-((5-(trifluoromethyl)pyridin-2-yl)amino)phenyl)acrylamide,
5) N-(2-methoxy-5-(pyridin-2-yl)-4-((5-(trifluoromethyl)pyridin-2-yl)amino)phenyl)acrylamide,
6) N-(3-(1-methyl-1H-imidazol-4-yl)-4-((5-(trifluoromethyl)pyridin-2-yl)amino)phenyl)acrylamide,
7) N-(4-((3-chloro-5-(trifluoromethyl)pyridin-2-yl)amino)-3-(pyridin-2-yl)phenyl)acrylam ide,
8) N-(4-((4,5-dichloropyridin-2-yl)amino)-3-(pyridin-2-yl)phenyl)acrylam ide,
9) N-(4-((3-chloro-5-(trifluoromethyl)pyridin-2-yl)amino)-3-(1-methyl-1H-imidazol-4-yl)phenyl)acrylamide ,
10) N-(4-((4,5-dichloropyridin-2-yl)amino)-3-(1-methyl-1H-imidazol-4-yl)phenyl)acrylam ide,
11) N-(6-((4-fluorobenzyl)amino)-[1,1'-biphenyl]-3-yl)acrylamide,
12) N-(4-((4-fluorobenzyl)amino)-3-(pyridin-2-yl)phenyl)acrylamide,
13) N-(3-(5-chloropyridin-2-yl)-4-((5-(trifluoromethyl)pyridin-2-yl)amino)phenyl)acrylamide,
14) N-(4-(pyridin-2-yl)-3-((4-(trifluoromethyl)phenyl)amino)phenyl)acrylamide,
15) N-(3-(pyrimidin-4-yl)-4-((5-(trifluoromethyl)pyridin-2-yl)amino)phenyl)acrylamide,
16) N-(4-((4,5-dichloropyridin-2-yl)amino)-3-(pyrimidin-4-yl)phenyl)acrylam ide,
17) N-(3-(pyridin-4-yl)-4-((5-(trifluoromethyl)pyridin-2-yl)amino)phenyl)acrylamide,
18) N-(4-((4,5-dichloropyridin-2-yl)amino)-3-(pyridin-4-yl)phenyl)acrylam ide,
19) N-(4-(5-chloro-4-fluoro-1H-indol-1-yl)-3-(pyridin-2-yl)phenyl)acrylam ide,
20) N-(4-((5-cyclopropylpyridin-2-yl)amino)-3-(pyridin-2-yl)phenyl)acrylam ide,
21) N-(4-((3-fluoro-5-(trifluoromethyl)pyridin-2-yl)amino)-3-(pyridin-2-yl)phenyl)acrylam ide,
22) N-(6-(((1r,4r)-4-hydroxycyclohexyl)amino)-5-phenylpyridin-3-yl)acrylamide,
23) (E)-N-(3-(4-fluorostyryl)-4-((2-(thiazol-2-yl)ethyl)amino)phenyl)acrylamide,
24) (E)-N-(3-(4-fluorostyryl)-4-((2-(thiazol-2-yl)ethyl)amino)phenyl)ethanesulfonamide,
25) N-(4-(cyclohexylamino)-3-(pyridin-3-yl)phenyl)acrylamide,
26) N-(4-(cyclopentylamino)-3-(pyridin-2-yl)phenyl)acrylamide,
27) (E)-N-(5-(4-fluorostyryl)-6-((2-(thiazol-2-yl)ethyl)amino)pyridin-3-yl)acrylamide,
28) (E)-N-(5-(4-fluorostyryl)-6-((2-(thiazol-2-yl)ethyl)amino)pyridin-3-yl)ethanesulfonamide,
29) N-(3-fluoro-5-(pyridin-2-yl)-4-((5-(trifluoromethyl)pyridin-2-yl)amino)phenyl)acrylamide,
30) N-(4-((5-chloro-4-cyclopropylpyridin-2-yl)amino)-3-(pyridin-2-yl)phenyl)acrylamide,
31) N-(3-(1-methyl-1H-imidazol-4-yl)-4-((4-(trifluoromethyl)pyridin-2-yl)amino)phenyl)acrylamide,
32) (E)-4-(dimethylamino)-N-(3-(1-methyl-1H-pyrazol-3-yl)-4-((4-(trifluoromethyl)pyridin-2-yl)amino)phenyl)-2-butenamide,
33) N-(4-((5-isopropylpyridin-2-yl)amino)-3-(pyridin-2-yl)phenyl)acrylam ide,
34) N-(4-((5-ethynylpyridin-2-yl)amino)-3-(1-methyl-1H-pyrazol-3-yl)phenyl)acrylamide,
35) N-(3-(1-methyl-1H-imidazol-4-yl)-4-((4-(trifluoromethyl)phenyl)amino)phenyl)acrylamide,
36) N-(4-((3-fluoro-5-(trifluoromethyl)pyridin-2-yl)amino)-3-(1-methyl-1Himidazol-4-yl)phenyl)acrylamide,
37) N-(4-((4-chloro-5-(trifluoromethyl)pyridin-2-yl)amino)-3-(1-methyl-1H-pyrazol-3-yl)phenyl)acrylamide,
38) N-(3-fluoro-5-(1-methyl-1H-imidazol-4-yl)-4-((5-(trifluoromethyl)pyridin-2-yl)amino)phenyl)acrylamide,
39) N-(2'-((5-(trifluoromethyl)pyridin-2-yl)amino)-[2,3'-bipyridin]-5'-yl)acrylamide,
40) N-(4-((3-fluoro-4-(trifluoromethyl)phenyl)amino)-3-(1-methyl-1Himidazol-4-yl)phenyl)acrylamide,
41) N-(5-(1-methyl-1H-imidazol-4-yl)-6-((4-(trifluoromethyl)phenyl)amino)pyridin-3-yl)acrylamide,
42) N-(3-(1-methyl-1H-pyrazol-3-yl)-4-((4-(trifluoromethyl)phenyl)amino)phenyl)acrylamide,
43) N-(4-((3-chloro-4-(trifluoromethyl)phenyl)amino)-3-(1 -methyl-1Himidazol-4-yl)phenyl)acrylamide,
44) N-(4-((2,3-difluoro-4-(trifluoromethyl)phenyl)amino)-3-(1 -methyl-1Himidazol-4-yl)phenyl)acrylamide,
45) N-(3-(1-methyl-1H-imidazol-4-yl)-4-((4-(trifluoromethoxy)phenyl)amino)phenyl)acrylamide,
46) N-(4-((3,5-difluoro-4-(trifluoromethyl)phenyl)amino)-3-(1-methyl-1Himidazol-4-yl)phenyl)acrylamide,
47) N-(4-((4-chlorophenyl)amino)-3-(1-methyl-1H-imidazol-4-yl)phenyl)acrylam ide,
48) N-(4-((5-bromo-6-methylpyridin-2-yl)amino)-3-(1-methyl-1H-imidazol-4-yl)phenyl)acrylamide,
49) N-(4-((5-chloro-6-methylpyridin-2-yl)amino)-3-(1-methyl-1H-imidazol-4-yl)phenyl)acrylamide,
50) N-(4-((5-chloropyridin-2-yl)amino)-3-(1-methyl-1H-imidazol-4-yl)phenyl)acrylamide,
51) N-(4-((5-bromo-4-chloropyridin-2-yl)amino)-3-(1-methyl-1H-imidazol-4-yl)phenyl)acrylamide,
52) N-(4-((5-bromopyridin-2-yl)amino)-3-(1-methyl-1H-imidazol-4-yl)phenyl)acrylam ide,
53) N-(4-((3-chloro-5-(trifluoromethyl)pyridin-2-yl)amino)-3-(1-methyl-1H-pyrazol-3-yl)phenyl)acrylamide,
54) N-(3-(1-methyl-1H-pyrazol-3-yl)-4-((4-(trifluoromethyl)pyridin-2-yl)amino)phenyl)acrylamide,
55) N-(4-((4,5-dichloropyridin-2-yl)amino)-3-(1-methyl-1H-pyrazol-3-yl)phenyl)acrylamide, and
56) N-(4-((3-fluoro-5-(trifluoromethyl)pyridin-2-yl)amino)-3-(1 -methyl-1H-pyrazol-3-yl)phenyl)acrylam ide.

14. A pharmaceutical composition for preventing or treating cancer or tumors, comprising the compound according to any one of claims 1 to 13, or a pharmaceutically acceptable salt thereof as an active ingredient.
